# EUROPÄISCHE PATENTSCHRIFT

(11) **EP 2 533 715 B1**
(45) Veröffentlichungstag und Bekanntmachung des Hinweises auf die Patenterteilung: **06.12.2017**
(21) Anmeldenummer: 11703439.7
(22) Anmeldetag: 08.02.2011
(51) Int. Cl.: A61B 17/70

(54) **VERBINDUNGSELEMENT FÜR EIN WIRBELSÄULENSTABILISIERUNGSSYSTEM UND WIRBELSÄULENSTABILISIERUNGSSYSTEM**
CONNECTING ELEMENT FOR A VERTEBRAL COLUMN-STABILIZING SYSTEM, AND VERTEBRAL COLUMN-STABILIZING SYSTEM
ÉLÉMENT DE LIAISON POUR UN SYSTÈME DE STABILISATION DE LA COLONNE VERTÉBRALE ET SYSTÈME DE STABILISATION DE LA COLONNE VERTÉBRALE

(30) Priorität: 08.02.2010 DE 102010000339
(43) Veröffentlichungstag der Anmeldung: 19.12.2012
(73) Patentinhaber: Aesculap AG, 78532 Tuttlingen (DE)
(72) Erfinder: BEGER, Jens, 78532 Tuttlingen (DE); CELMEROWSKI, Beate, 78594 Gunningen (DE); MAAS, Allan, 78465 Konstanz (DE)
(74) Vertreter: Hoeger, Stellrecht & Partner Patentanwälte mbB
(86) Internationale Anmeldenummer: PCT/EP2011/051838
(87) Internationale Veröffentlichungsnummer: WO 2011/095641

(56) Entgegenhaltungen:
- WO-A1-2006/101737
- WO-A1-2007/090015
- WO-A1-2011/038141
- US-A1- 2008 221 681
- US-A1- 2008 234 736
- US-A1- 2009 326 584

## Beschreibung

Die vorliegende Erfindung betrifft ein Verbindungselement für ein Wirbelsäulenstabilisierungssystem mit einem ersten Befestigungsabschnitt zum Festlegen an einer ersten Knochenbefestigungseinrichtung, einem zweiten Befestigungsabschnitt zum Festlegen an einer zweiten Knochenbefestigungseinrichtung und einem zwischen dem ersten und dem zweiten Befestigungsabschnitt angeordneten oder ausgebildeten, mindestens teilweise flexiblen Zwischenabschnitt, welcher Zwischenabschnitt mindestens eine in einer Richtung quer zu einer vom Zwischenabschnitt definierten Längsachse seitlich geöffnete Ausnehmung aufweist, wobei mindestens ein Bewegungsbegrenzungsglied vorgesehen ist, welches einen Füllkörper und mindestens eine parallel oder im Wesentlichen parallel zur Längsachse wirkende Anschlageinrichtung umfasst, und wobei der Füllkörper in einer Grundstellung, in welcher auf den Zwischenabschnitt keine äußeren Kräfte wirken, in die mindestens eine Ausnehmung mindestens teilweise eingreift.

Ferner betrifft die vorliegende Erfindung ein Wirbelsäulenstabilisierungssystem umfassend mindestens eine erste Knochenbefestigungseinrichtung, mindestens eine zweite Knochenbefestigungseinrichtung und ein Verbindungselement, welches Verbindungselement einen ersten Befestigungsabschnitt zum Festlegen an der mindestens einen ersten Knochenbefestigungseinrichtung, einen zweiten Befestigungsabschnitt zum Festlegen an der mindestens einen zweiten Knochenbefestigungseinrichtung und einen zwischen dem ersten und dem zweiten Befestigungsabschnitt angeordneten oder ausgebildeten, mindestens teilweise flexiblen Zwischenabschnitt umfasst, welcher Zwischenabschnitt mindestens eine quer zu einer vom Zwischenabschnitt definierten Längsachse seitliche geöffnete Ausnehmung aufweist, wobei mindestens ein Bewegungsbegrenzungsglied vorgesehen ist, welches einen Füllkörper und mindestens eine parallel oder im Wesentlichen parallel zur Längsachse wirkende Anschlageinrichtung umfasst, und wobei der Füllkörper in einer Grundstellung, in welcher auf den Zwischenabschnitt keine äußeren Kräfte wirken, in die mindestens eine Ausnehmung mindestens teilweise eingreift.

Sowohl ein Verbindungselement als auch ein Wirbelsäulenstabilisierungssystem sind beispielsweise aus der US 2006/0184171 A1 bekannt. Das darin offenbarte Verbindungselement weist einen Zwischenabschnitt in Form eines schleifenförmig gewundenen Stabes auf, wobei die Schleifen entlang einer Verbindungsachse von einem ersten zu einem zweiten Ende des Verbindungselements abwechselnd auf zwei entgegengesetzten Seiten der Verbindungsachse verlaufen.

Eine Steifigkeit des Zwischenabschnitts und damit des Verbindungselements insgesamt wird definiert durch die Ausgestaltung des Zwischenabschnitts. Eine Änderung der Steifigkeit oder anderer mechanischer Eigenschaften des Zwischenabschnitts ist damit insbesondere nach einer Implantation nicht mehr möglich. Falls Änderungen gewünscht sind, muss das Verbindungselement komplett ausgetauscht werden. In Abhängigkeit der Physiologie eines Patienten müssen bei den bekannten Systemen eine Vielzahl von Verbindungselementen mit unterschiedlichen Steifigkeiten und gegebenenfalls Abmessungen bereitgehalten werden, um einem Operateur die Auswahl des am besten geeigneten Verbindungselements unter Berücksichtigung des Befunds des Patienten zu gestatten.

Weitere Verbindungselemente und Wirbelsäulenstabilisierungssysteme sind aus der WO 2007/090015 A1, der US 2008/0234736 A1, der WO 2006/101737 A1, der US 2008/0221681 A1, der US 2009/0326584 A1 sowie der WO 2011/038141 A1 bekannt.

Es ist daher Aufgabe der vorliegenden Erfindung, ein Verbindungselement und ein Wirbelsäulenstabilisierungssystem der eingangs beschriebenen Art so zu verbessern, dass mindestens eine mechanische Eigenschaft des Verbindungselements, insbesondere des Zwischenabschnitts desselben, auf einfache und sichere Weise individuell einstellbar ist.

Diese Aufgabe wird sowohl bei einem Verbindungselement als auch bei einem Wirbelsäulenstabilisierungssystem der eingangs beschriebenen Art erfindungsgemäß dadurch gelöst, das mindestens eine Bewegungsbegrenzungsglied eine in Umfangsrichtung wirkende Anschlageinrichtung umfasst. Vorteilhafte Weiterbildungen der Erfindung sind Gegenstand der abhängigen Ansprüche.

Das mindestens eine Bewegungsbegrenzungsglied, das in der beschriebenen Weise mit dem Zwischenabschnitt in Eingriff steht, ermöglicht die Begrenzung einer Beweglichkeit des Zwischenabschnitts in definierter Weise. Insbesondere können je nach Ausgestaltung des Bewegungsbegrenzungsglieds und in Abhängigkeit der Anzahl der Ausnehmungen sowie der Bewegungsbegrenzungsglieder wahlweise eine Kompression, eine Flexion sowie eine Rotation oder Verwindung des Zwischenabschnitts zugelassen, eingeschränkt oder ganz ausgeschlossen werden. Mit anderen Worten bedeutet dies, dass in Abhängigkeit der Ausgestaltung des Bewegungsbegrenzungsglieds, insbesondere der Anschlageinrichtung, mechanische Eigenschaften des Verbindungselements, insbesondere des Zwischenabschnitts, gezielt eingestellt werden können. Ein Operateur hat so die Möglichkeit, noch während eines chirurgischen Eingriffs ein Standardverbindungselement mit einem mindestens teilweise flexiblen Zwischenabschnitt auszuwählen und durch Anbringen eines oder mehrerer Bewegungsbegrenzungsglieder die mechanischen Eigenschaften an die für den jeweiligen Fall erforderlichen Bedürfnisse anzupassen. Insbesondere ist es günstig, wenn der Füllkörper in der Grundstellung spielbehaftet oder mit Spiel in die Ausnehmung eingreift. Auf diese Weise kann insbesondere noch eine gezielte Flexion oder Kompression des Zwischenabschnitts ermöglicht werden beziehungsweise durch den Füllkörper des Bewegungsbegrenzungsglieds begrenzt werden.

Vorteilhaft ist es, wenn die Anschlageinrichtung mindestens einen am Füllkörper angeordneten, ausgebildeten und/oder von diesem abstehenden Anschlag umfasst, welcher parallel oder im Wesentlichen parallel zur Längsachse und in Umfangsrichtung wirkt. Durch das Vorsehen mindestens eines Anschlags am Füllkörper in der beschriebenen Weise lässt sich eine Beweglichkeit des Zwischenabschnitts einfach und sicher beschränken oder ausschalten. Insbesondere ist diese Ausgestaltung günstig, wenn zwei, drei oder mehr Bewegungsbegrenzungsglieder jeweils in eine Ausnehmung des Zwischenabschnitts eingreifend vorgesehen sind. Der mindestens eine Anschlag kann insbesondere Anschlagflächen aufweisen, welche in Richtung oder im Wesentlichen in Richtung der Längsachse oder in Umfangsrichtung weisen.

Besonders einfach herstellen lässt sich das Verbindungselement, wenn mehrere Ausnehmungen vorgesehen sind, die alternierend in entgegengesetzte Richtungen weisend geöffnet sind. Beispielsweise durch eine schlangenförmige oder zick-zack-förmige Verformung eines Stabs oder streifenförmigen Materials lässt sich eine solche Ausgestaltung auf einfache Weise realisieren. Auch eine Herstellung durch spanende Bearbeitung aus einem Vollmaterial ist denkbar. Durch die Ausbildung benachbarter, quasi um 180° bezogen auf die Längsachse gedrehter Ausnehmungen kann eine Flexion des Zwischenabschnitts in entgegengesetzte Richtungen bei Bedarf auch gezielt unterschiedlich durch entsprechend dafür gestaltete Bewegungsbegrenzungsglieder beeinflusst werden.

Um eine möglichst kontinuierliche Einflussnahme auf den Zwischenabschnitt und seine mechanische Eigenschaften zu ermöglichen, ist es günstig, wenn jeder Ausnehmung ein Bewegungsbegrenzungsglied zugeordnet ist. Prinzipiell ist es möglich, auch Bewegungsbegrenzungsglieder derart auszubilden und vorzusehen, die zwei Füllkörper aufweisen, um benachbarte oder übernächste Nachbarn von Ausnehmungen miteinander zu verbinden oder zu koppeln. Beispielsweise kann ein Bewegungsbegrenzungsglied auch alle in dieselbe Richtung weisend geöffneten Ausnehmungen durch eine entsprechende Zahl von Füllkörpern ganz oder teilweise ausfüllen.
Besonders einfach in der Herstellung sowie in der Montage wird das Verbindungselement, wenn alle Bewegungsbegrenzungsglieder identisch ausgebildet sind. Auf diese Weise können bei einer Montage des Verbindungselements keine Verwechslungen auftreten.

Gemäß einer weiteren Ausführungsform kann vorgesehen sein, dass Bewegungsbegrenzungsglieder in benachbarte Ausnehmungen relativ zueinander um 180° um die Längsachse gedreht eingesetzt sind. Insbesondere kann dies der Fall sein bei identisch ausgebildeten Bewegungsbegrenzungsgliedern. Des Weiteren ist dies vorteilhaft, wenn jeder Ausnehmung genau ein Bewegungsbegrenzungsglied zugeordnet ist und wenn benachbarte Ausnehmungen in entgegengesetzte Richtungen weisend geöffnet sind.

Vorteilhaft ist es, wenn Anschlageinrichtungen benachbarter Bewegungsbegrenzungsglieder in der Grundstellung mindestens teilweise kontaktlos einander gegenüberliegen und in einer aus der Grundstellung ausgelenkten Stellung aneinander anliegen. Beispielsweise können die in der Grundstellung einander gegenüberliegenden Anschlageinrichtungen durch einen Spalt voneinander getrennt oder voneinander beabstandet sein. Durch eine Breite des Spalts kann so eine maximal mögliche Verformung des Zwischenelements, beispielsweise eine Kompression oder eine Flexion, gezielt eingestellt werden. Eine Verformung wird dadurch begrenzt, dass in der aus der Grundstellung ausgelenkten Stellung die Anschlageinrichtungen aneinander anliegen und eine weitere Bewegung derselben, beispielsweise aufeinander zu, nicht mehr möglich ist.

Auf einfache Weise lassen sich Anschlageinrichtungen in der beschriebenen Weise ausbilden, wenn die parallel oder im Wesentlichen parallel zur Längsachse wirkenden Anschläge in der Grundstellung voneinander beabstandet sind und in der ausgelenkten Stellung aneinander anstoßen. Diese Ausgestaltung kann sowohl bei in Längsrichtung als auch bei in Umfangsrichtung wirkenden Anschlägen realisiert werden, um gezielt insbesondere Kompressionsbewegungen und Rotationsbewegungen, zum Beispiel Verwindungen, des Zwischenabschnitts in einem bestimmten Bereich zuzulassen und darüber hinaus zu beschränken.

Vorzugsweise greifen die in Umfangsrichtung wirkenden Anschläge in der Grundstellung und/oder in der ausgelenkten Stellung formschlüssig oder im Wesentlichen formschlüssig ineinander. Durch auf diese Weise ausgebildete Formschlüsse kann insbesondere eine Rotations- oder Verwindungsbewegung benachbarter Ausnehmungen des Zwischenabschnitts durch zusammenwirkende Bewegungsbegrenzungsglieder vollständig oder im Wesentlichen vollständig unterbunden werden. So lässt sich beispielsweise auf einfache Weise eine Rotationssicherungs- oder Verwindungsverhinderungseinrichtung ausbilden.

Auf besonders einfache Weise ausbilden lässt sich die Anschlageinrichtung, wenn jedes Bewegungsbegrenzungsglied eine Anschlageinrichtung mit zwei in entgegengesetzte Richtungen weisenden Anschlägen umfasst. Die Anschläge können insbesondere gebildet werden durch Seitenflächen oder Flächenbereiche definierende Anschlagflächen am Füllkörper oder an vom Füllkörper abstehenden Vorsprüngen oder Ausnehmungen derselben.

Besonders einfach wird der Aufbau des Verbindungselements und damit dessen Konstruktion und Herstellung, wenn das mindestens eine Bewegungsbegrenzungsglied und/oder der Zwischenabschnitt spiegelsymmetrisch zu einer die Längsachse enthaltenden Spiegelebene ausgebildet sind.

Vorteilhaft kann es ferner sein, wenn das Verbindungselement eine Antiverwindungseinrichtung zum Verhindern einer Verwindung des Zwischenabschnitts um die Längsachse umfasst. Die Antiverwindungseinrichtung ermöglicht es insbesondere, Scherkräfte, die quer zur Längsachse und/oder in Umfangsrichtung wirken, aufzunehmen und das Verbindungselement zu stabilisieren.

Besonders günstig ist es, wenn die Antiverwindungseinrichtung den mindestens einen in Umfangsrichtung wirkenden Anschlag umfasst. Selbstverständlich kann die Antiverwindungseinrichtung auch zwei oder mehr in Umfangsrichtung wirkende Anschläge umfassen, die wiederum durch Anschlagflächen definiert oder gebildet werden können. Derartige Anschläge wirken vorzugsweise Scher- oder Verwindungskräften direkt entgegen.

Gemäß einer weiteren Ausführungsform kann vorgesehen sein, dass die Antiverwindungseinrichtung mindestens eine Kupplungseinrichtung umfasst zum Kuppeln benachbarter Bewegungsbegrenzungsglieder, deren Füllkörper in benachbarte Ausnehmungen des Zwischenabschnitts eingreifen. Auf diese Weise können durch die Ausnehmungen begrenzte Bereiche oder Abschnitte des Zwischenabschnitts über die Antiverwindungseinrichtung direkt oder indirekt miteinander gekoppelt und ein Verwinden des Zwischenabschnitts eingeschränkt oder gar unterbunden werden.

Besonders einfach ausbilden lässt sich die mindestens eine Kupplungseinrichtung, wenn sie erste und zweite Kupplungsglieder umfasst, welche kraft- und/oder formschlüssig in Eingriff stehen, und wenn ein erstes Kupplungsglied eines Bewegungsbegrenzungsglieds in Richtung auf das zweite Kupplungsglied eines mit einer benachbarten Ausnehmung mindestens teilweise in Eingriff stehenden Bewegungsbegrenzungsglieds hin weist. Durch die derart ausgebildeten Kupplungsglieder lassen sich insbesondere benachbarte Bewegungsbegrenzungsglieder oder auch übernächste oder gegebenenfalls noch weiter entfernte Nachbarn auf einfache Weise miteinander koppeln.

Vorteilhaft ist es, wenn dass mindestens eine Bewegungsbegrenzungsglied mindestens ein erstes und mindestens ein zweites Kupplungsglied umfasst, die jeweils einer Kupplungseinrichtung zugeordnet sind, und wenn das mindestens eine erste und das mindestens eine zweite Kupplungsglied in die entgegengesetzte Richtungen weisend ausgebildet oder angeordnet sind. Die ersten und zweiten Kupplungsglieder eines Bewegungsbegrenzungsglieds bilden somit jeweils einen Teil einer Kupplungseinrichtung. Mit anderen Worten ist in diesem Fall jedes Bewegungsbegrenzungsglied mindestens teilweise an zwei Kupplungseinrichtungen zum Koppeln des Bewegungsbegrenzungsglieds mit zwei weiteren Bewegungsbegrenzungsgliedern beteiligt.

Besonders einfach wird der Aufbau des Verbindungselements, wenn die ersten und zweiten Kupplungsglieder die in Umfangsrichtung wirkenden Anschläge umfassen oder bilden. Insbesondere können diese in Umfangsrichtung oder zumindest in einer Richtung quer zur Längsachse weisende Anschlagflächen umfassen.

Auf einfache Weise lassen sich insbesondere benachbarte Bewegungsbegrenzungsglieder miteinander koppeln, wenn das mindestens eine erste und das mindestens eine zweite Kupplungsglied parallel zur Längsachse ausgerichtet sind. Die Kupplungsglieder können insbesondere in Form von Vorsprüngen und/oder Ausnehmungen ausgebildet sein, die kraft- und/oder formschlüssig in einer Kupplungsstellung miteinander in Eingriff stehen können.

Günstig ist es, wenn die mindestens eine Ausnehmung in der Grundstellung eine quer zur Längsachse und von dieser weg weisende Einführöffnung aufweist zum Einführen des Füllkörpers in einer Richtung quer zur Längsachse und auf diese hin. Eine Montage des Verbindungselements wird so besonders einfach, denn die Füllkörper der Bewegungsbegrenzungsglieder können so einfach von der Seite her kommend in Richtung auf die Längsachse hin in die Ausnehmungen eingeschoben werden.

Vorzugsweise bildet die Einführöffnung eine Engstelle. Durch entsprechende Ausgestaltung des Füllkörpers, welcher beispielsweise eine zur Engstelle korrespondierende Einschnürung oder Querschnittsverjüngung aufweisen kann, lässt sich das Bewegungsbegrenzungsglied durch in Eingriff Bringen des Füllkörpers und der Ausnehmung ohne weitere Hilfsmittel am Zwischenabschnitt unverlierbar sichern.

Auf einfache und sichere Weise kann das Bewegungsbegrenzungsglied am Zwischenabschnitt gehalten werden, wenn sich die Ausnehmung im Inneren ausgehend von der Engstelle erweitert. So kann beispielsweise ein Füllkörper selbst dann in der Ausnehmung gehalten werden, wenn er diese nicht vollständig, also beispielsweise spielbehaftet, ausfüllt, jedoch einen maximalen Querschnitt beziehungsweise eine Querschnittsfläche aufweist, die größer als eine von der Einführöffnung definierte Querschnittsfläche ist. Die beschriebenen Varianten gelten insbesondere für die Grundstellung, wenn keine äußeren Kräfte auf das Verbindungselement, insbesondere auf dessen Zwischenabschnitt, wirken. Beispielsweise zum Einführen der Füllkörper in die Ausnehmungen kann es auch günstig sein, wenn der Zwischenabschnitt derart verformt wird, dass sich die jeweilige Einführöffnung zum Einführen des Füllkörpers des Bewegungsbegrenzungsglieds aufweitet.

Um ein möglichst kompaktes Verbindungselement auszubilden und die Ausnehmung zu schützen, ist es vorteilhaft, wenn das Bewegungsbegrenzungsglied ein Schließelement zum Verschließen der Einführöffnung umfasst. Besonders einfach herstellen lässt sich das Bewegungsbegrenzungsglied, wenn das Schließelement am Füllkörper angeordnet oder ausgebildet ist.

Die Stabilität des Bewegungsbegrenzungsglieds lässt sich insbesondere dadurch erhöhen, dass die mindestens eine Anschlageinrichtung das Schließelement umfasst. Beispielsweise können äußere Seitenflächen oder Kanten des Schließelements Anschläge oder Anschlagflächen der Anschlageinrichtung ausbilden. Das Schließelement kann so zumindest eine Doppelfunktion übernehmen.

Gemäß einer weiteren Ausführungsform kann vorgesehen sein, dass Schließelemente von übernächsten Nachbarn von Bewegungsbegrenzungsgliedern in der Grundstellung aneinander anliegen oder durch einen Spalt voneinander beabstandet sind. Insbesondere bei alternierend in entgegengesetzte Richtungen geöffnete benachbarte Ausnehmungen können so übernächste Nachbarn von Ausnehmungen durch mit diesen in Eingriff stehende Bewegungsbegrenzungsglieder miteinander gekoppelt werden, um beispielsweise eine Begrenzung einer Kompressions- oder einer Flexionsbewegung zu bewirken.

Die Herstellung des Zwischenabschnitts wird besonders einfach, wenn die mindestens eine Ausnehmung in zwei voneinander entgegengesetzte Richtungen weisend seitlich geöffnet ist und wenn das mindestens eine Bewegungsbegrenzungsglied die mindestens eine Ausnehmung mindestens einseitig teilweise verschließt. Insbesondere wenn der Zwischenabschnitt aus einem Vollmaterial durch spanende Bearbeitung hergestellt ist, kann das die Ausnehmung mindestens teilweise verschließende Bewegungsbegrenzungsglied ein unbeabsichtigtes Lösen des Füllkörpers aus der Ausnehmung verhindern.

Um das mindestens eine Bewegungsbegrenzungsglied in definierter Weise in einer Ausnehmung zu positionieren, ist es günstig, wenn es die mindestens eine Ausnehmung beidseitig jeweils vollständig oder im Wesentlichen vollständig verschließt. Auf diese Weise kann insbesondere eine Bewegung in einer Richtung sowohl quer zur Längsachse als auch quer zu einer von der Einführöffnung definierten Einführrichtung des mindestens einen Bewegungsbegrenzungsglieds relativ zur Ausnehmung unterbunden werden.

Auf einfache Weise lässt sich die seitlich geöffnete Ausnehmung seitlich verschließen, wenn das mindestens eine Bewegungsbegrenzungsglied mindestens ein seitliches Verschlusselement umfasst zum mindestens teilweisen seitlichen Verschließen der mindestens einen Ausnehmung.

Besonders sicher und definiert lässt sich das mindestens eine Bewegungsbegrenzungsglied an mindestens einer Ausnehmung anordnen, wenn es zwei seitliche Verschlusselemente aufweist zum Verschließen der mindestens einen Ausnehmung auf zwei einander entgegengesetzten Seiten. Wie bereits dargelegt, kann durch die beiden Verschlusselemente eine Bewegung des Füllkörpers quer zur Längsachse und quer zur Einführrichtung unterbunden werden.

Auf einfache Weise herstellen lässt sich das mindestens eine Bewegungsbegrenzungsglied, wenn das mindestens eine seitliche Verschlusselement seitlich vom Füllkörper abstehend ausgebildet ist. Insbesondere kann es in Richtungen parallel zur Längsachse über den Füllkörper vorstehend ausgebildet sein.

Vorteilhaft ist es, wenn das mindestens eine seitliche Verschlusselement mindestens einen der parallel oder im Wesentlichen parallel zur Längsachse oder in Umfangsrichtung wirkenden Anschläge umfasst oder trägt. Das Bewegungsbegrenzungsglied und damit das Verbindungselement insgesamt lassen sich so besonders kompakt ausbilden. Das seitliche Verschlusselement kann somit mehrere Funktionen gleichzeitig übernehmen.

Vorzugsweise weist der Füllkörper mindestens eine Querschnittsverjüngung auf. Insbesondere bei einer verengten Einführöffnung kann so ohne weitere Hilfsmittel erreicht werden, dass das mindestens eine Bewegungsbegrenzungsglied zumindest in der Grundstellung mit dem Zwischenabschnitt in Eingriff gebracht werden kann, ohne sich in unbeabsichtigter Weise von diesem lösen zu können.

Um Abstoßungsreaktionen zu verhindern, ist es günstig, wenn das mindestens eine Bewegungsbegrenzungsglied aus einem biokompatiblen Material hergestellt ist.

Besonders kostengünstig hergestellt werden kann das mindestens eine Bewegungsbegrenzungsglied, wenn das biokompatible Material ein Kunststoff ist. Vorzugsweise ist oder enthält der Kunststoff Polyethylen, Polyetheretherketon, Polyurethan, Polycarbonaturethan oder mindestens einen faserverstärkenden Kunststoff.

Die Herstellung des mindestens einen Bewegungsbegrenzungsglieds lässt sich weiter vereinfachen, wenn es einstückig ausgebildet ist. Beispielsweise lässt es sich so durch Spritzen herstellen.

Einfach und kostengünstig herstellen lässt sich das mindestens eine Bewegungsbegrenzungsglied, wenn es in Form eines Spritzgussteils ausgebildet ist. Beispielsweise kann es sich um ein Kunststoffspritzgussteil handeln.

Gemäß einer weiteren Ausführungsform kann vorgesehen sein, dass mehrere mit dem Zwischenabschnitt in Eingriff stehende Bewegungsbegrenzungsglieder vorgesehen sind, die eine gemeinsame und im Wesentlichen zylindrische Hüllkontur definieren. Auf diese Weise kann eine Verletzung von umliegendem Körpergewebe im Wesentlichen verhindert werden. Insbesondere durch die beschriebenen Schließ- und Verschlusselemente ist es für das Verbindungselement umgebendes Körpergewebe praktisch nicht möglich, in die Ausnehmungen des Zwischenelements einzudringen und in diesen eingeklemmt zu werden. Durch die zylindrische Hüllkontur kann insbesondere ein im Wesentlichen stabförmiges Design des Verbindungselements realisiert werden.

Um eine Grundflexibilität des Zwischenabschnitts zu ermöglichen, ist es günstig, wenn dieser mindestens drei Ausnehmungen umfasst. Selbstverständlich kann er auch vier, fünf oder mehr Ausnehmungen umfassen. Typischerweise kann eine Flexibilität des Zwischenabschnitts durch Erhöhung der Zahl der Ausnehmungen erhöht werden.

Vorzugsweise ist der Zwischenabschnitt elastisch ausgebildet. Zum einen kann auf diese Weise eine Bruchgefahr des Verbindungselements verringert werden. Zum anderen kann die Elastizität des Zwischenabschnitts genutzt werden, um das Verbindungselement selbsttätig, das heißt automatisch, nach einer Auslenkung wieder in die Grundstellung zurück zu überführen.

Vorteilhaft ist es, wenn das Verbindungselement eine Rückstelleinrichtung zum automatischen Rückführen des Verbindungselements aus einer aus der Grundstellung ausgelenkten Auslenkstellung in die Grundstellung zurück umfasst. Durch die Rückstelleinrichtung können zum Verformen des Verbindungselements, insbesondere des Zwischenabschnitts, erforderliche Kräfte vordefiniert werden. Aufgrund der Rückstelleinrichtung werden umso höhere Kräfte benötigt, je weiter das Verbindungselement aus der Grundstellung ausgelenkt werden soll.

Besonders einfach herstellen lässt sich das Verbindungselement, wenn die Rückstelleinrichtung mindestens ein Rückstellelement umfasst zum automatischen Rückführen des Verbindungselements aus der Auslenkstellung in die Grundstellung zurück.

Günstig ist es, wenn das Rückstellelement in Form eines Federelements ausgebildet ist. Insbesondere kann das Rückstellelement in Form einer Blattfeder ausgebildet sein. Des Weiteren denkbar sind mehrere Rückstellelemente, die einzelne Abschnitte des Zwischenabschnitts miteinander koppeln. Insbesondere wenn die letztgenannten Abschnitte des Zwischenabschnitts starr und unflexibel sind, kann so durch das Vorsehen der Rückstellelemente eine Flexibilität des Zwischenabschnitts in definierter Weise vorgegeben werden.

Ein besonders kompaktes Verbindungselement lässt sich ausbilden, wenn der Zwischenabschnitt das Rückstellelement bildet oder umfasst. Beispielsweise kann der Zwischenabschnitt in Form einer schlangenförmig gewundenen Blattfeder ausgebildet sein, welche so im Querschnitt im Wesentlichen tropfenförmige Ausnehmungen definiert, wobei benachbarte Ausnehmungen jeweils in entgegengesetzte Richtungen und von der Längsachse weg weisend geöffnet sind.

Vorteilhaft ist es, wenn der Zwischenabschnitt aus einem streifen- oder stabförmigen Material schlangen- oder zick-zack-förmig gewunden ist. Beispielsweise kann der Zwischenabschnitt so aus einer Blattfeder einfach hergestellt werden. Alternativ kann er jedoch auch aus einem Vollmaterial hergestellt sein, beispielsweise durch Fräsen oder Erodieren. Insbesondere die Herstellung aus einem Vollmaterial ermöglicht es, das Verbindungselement insgesamt einstückig auszubilden. Werden die Befestigungsabschnitte und der Zwischenabschnitt getrennt voneinander hergestellt, können die Teile insbesondere durch Schweißen miteinander verbunden werden.

Günstig ist es, wenn der Zwischenabschnitt eine Mehrzahl von gekrümmten Abschnitten aufweist, die sich von entgegengesetzten Seiten der Längsachse alternierend weg erstrecken. Dies ermöglicht es insbesondere, den Zwischenabschnitt schlangen- oder zick-zack-förmig auszubilden. Des Weiteren können die gekrümmten Abschnitte sowohl eine Flexibilität und, je nach Materialauswahl, auch eine gewünschte Elastizität des Zwischenabschnitts definieren.

Um die Gefahr von Verletzungen von umliegendem Gewebe zu verringern, ist es günstig, wenn die gekrümmten Abschnitte von der Längsachse weg weisend konvex gekrümmt sind. Sie können so im Wesentlichen frei von scharfen Kanten hergestellt werden.

Des Weiteren kann es vorteilhaft sein, wenn die gekrümmten Abschnitte über geradlinige oder ebene Abschnitte miteinander verbunden sind. Sie dienen insbesondere als Abstandshalter zwischen den gekrümmten Abschnitten und müssen nicht zwingend flexibel oder elastisch sein. Alternativ können jedoch auch die gekrümmten Abschnitte unflexibel oder inelastisch sein, die geradlinigen oder ebenen Abschnitte dagegen flexibel oder elastisch.

Um die Stabilität des Zwischenabschnitts zu erhöhen, ist es günstig, wenn die geradlinigen oder ebenen Abschnitte eine vom Zwischenabschnitt definierte Längsachse schneiden. Insbesondere kann so ein Abstand zwischen aufeinanderfolgenden gekrümmten Abschnitten in definierter Weise eingestellt werden. Durch die Abstände zwischen den gekrümmten, aufeinanderfolgenden Abschnitten kann insbesondere eine Beweglichkeit oder Flexibilität des Zwischenabschnitts definiert werden, insbesondere für eine Flexions- und/oder Extensionsbewegung der Befestigungsabschnitte des Verbindungselements relativ zueinander.

Um die maximalen Biegespannungen in einem gekrümmten oder bogenförmigen Abschnitt des Zwischenabschnitts zu verringern, ist es günstig, wenn eine Dicke des Zwischenabschnitts im Bereich der gekrümmten Abschnitte größer ist als im Bereich der ebenen Abschnitte.

Günstigerweise beträgt die Dicke der gekrümmten Abschnitte das etwa 1,1fache bis etwa 1,5fache einer Dicke der ebenen Abschnitte. Vorzugsweise beträgt die Dicke der gekrümmten Abschnitte das etwa 1,3fache bis etwa 1,35fache der Dicke der ebenen Abschnitte. Die angegebenen Bereiche eignen sich hervorragend, um maximal auftretende Biegespannungen im Bereich der gekrümmten Abschnitte zu minimieren. Insgesamt kann so eine Stabilität des Zwischenabschnitts und damit auch des gesamten Verbindungselements erhöht werden.

Vorteilhaft ist es, wenn eine Steifigkeit des Zwischenabschnitts einen Wert in einem Bereich von 30 N/mm bis etwa 150 N/mm aufweist. Die angegebene Steifigkeit des Zwischenabschnitts kann sich insbesondere auf dessen Grundform ohne angekoppelte Bewegungsbegrenzungsglieder beziehen. Die Steifigkeit des Zwischenabschnitts lässt sich mittels geeigneter Bewegungsbegrenzungsglieder insbesondere noch über den angegebenen Wert von etwa 150 N/mm erhöhen.

Besonders gute flexible beziehungsweise elastische Eigenschaften des Zwischenabschnitts lassen sich erreichen, wenn dieser aus einem flachen oder im Wesentlichen flachen Material gebildet ist. Beispielsweise kann er aus einem im Wesentlichen flachen Material gebildet sein, bei dem sich Abschnitte unterschiedlicher Dicke abwechseln, wobei der Zwischenabschnitt dann vorzugsweise so geformt wird, dass die dickeren Abschnitte gekrümmte Abschnitte des Zwischenabschnitts bilden, die dünneren Abschnitte des im Wesentlichen flachen Materials die gekrümmten Abschnitte verbindende ebene oder flache Abschnitte.

Günstig ist es, wenn der Zwischenabschnitt zwei voneinander weg weisende Seitenflächen umfasst, die in der Grundstellung parallel oder im Wesentlichen parallel zueinander verlaufen. Die Seitenflächen können wiederum bei einem schlangenförmig gewundenen Abschnitt ebenfalls schlangenförmig verlaufend ausgebildet sein. Die Seitenflächen können insbesondere als Anschläge für Verschlusselemente der Bewegungsbegrenzungsglieder dienen.

Besonders einfach wird die Herstellung des Verbindungselements, wenn die Seitenflächen in der Grundstellung parallel oder im Wesentlichen parallel zu einer die Längsachse enthaltenden Mittelebene verlaufen.

Um die Kompatibilität des Verbindungselements mit am Markt verfügbaren Wirbelsäulenstabilisierungssystemen zu erhöhen, ist es vorteilhaft, wenn der erste und/oder der zweite Befestigungsabschnitt stabförmig ausgebildet sind.

Vorzugsweise weisen der erste und/oder der zweite Befestigungsabschnitt einen kreisförmigen oder im Wesentlichen kreisförmigen Querschnitt auf. Das Verbindungselement kann so insbesondere anstelle eines rundstabförmigen Verbindungselements mit bekannten Wirbelsäulenstabilisierungssystemen kombiniert werden. Selbstverständlich sind auch ovale oder N-eckige Querschnitte möglich, insbesondere dreieckige, viereckige oder sechseckige Querschnitte, die korrespondierend zu entsprechenden Aufnahmen an Knochenbefestigungseinrichtungen ausgebildet sind.

Um Abstoßungsreaktionen zu vermeiden, ist es günstig, wenn der Zwischenabschnitt und/oder der erste Befestigungsabschnitt und/oder der zweite Befestigungsabschnitt aus einem körperverträglichen Material hergestellt sind.

Vorzugsweise ist oder enthält das körperverträgliche Material eine Titanlegierung, eine Kobalt-Chrom-Legierung, Polyetheretherketon oder kohlefaserverstärktes Polyetheretherketon. Insbesondere die genannten Materialien sind biokompatibel und als Implantatmaterialien hervorragend geeignet.

Vorteilhaft ist es, wenn das Verbindungselement eine mindestens teilweise durch ein Strahlverfahren bearbeitete äußere Oberfläche aufweist. Eine solche Ausgestaltung ist insbesondere auch vorteilhaft bei einem Verbindungselement der eingangs beschriebenen Art. Durch Bearbeitung der äußeren Oberfläche mittels eines Strahlverfahrens lässt sich insbesondere eine Dauerfestigkeit des Verbindungselements deutlich erhöhen, was insbesondere bei dauerhaft eingesetzten Implantaten von großem Vorteil ist.

Vorzugsweise ist die mindestens teilweise bearbeitete Oberfläche durch ein Kugelstrahlverfahren bearbeitet. Ein solches lässt sich besonders einfach durchführen. Gegebenenfalls lassen sich gezielt auch nur einzelne Bereiche der äußeren Oberfläche bearbeiten. Als Kugeln können insbesondere Stahl- oder Glaskugel zum Strahlen des Verbindungselements genutzt werden.

Günstig kann es ferner sein, wenn die mindestens teilweise bearbeitete Oberfläche durch ein "Laser-Peening"-Verfahren bearbeitet ist, bei dem die Oberfläche durch gepulste Laserstrahlbeaufschlagung erzeugte Schockwellen zur Erzeugung von Druckeigenspannungen ausgesetzt wurde. Wie auch beim Kugelstrahlverfahren können durch Beschuss mit einem Laser Druckeigenspannungen eingebracht werden. Diese wirken insbesondere Ermüdungsanrissen entgegen.

Der Herstellungsaufwand des Verbindungselements kann insbesondere dadurch verringert werden, dass die gesamte äußere Oberfläche vollständig bearbeitet ist. Auf dieses Weise kann auf eine Abdeckung einzelner Bereich der Oberfläche des Verbindungselements vor und während des Strahlens verzichtet werden.

Vorteilhaft ist, wenn die äußere Oberfläche bis auf die Seitenflächen des Zwischenabschnitts bearbeitet ist. Bei den Seitenflächen des Zwischenabschnitts handelt es sich insbesondere um zu einer die Längsachse enthaltenden Mittelebene parallel oder im Wesentlichen parallel verlaufende Seitenflächen. Vor dem Strahlbehandeln werden die Seitenflächen vorzugsweise abgedeckt. Auf diese Weise kann verhindert werden, dass insbesondere ein aus einem flachen Material gebildeter, dünnwandiger Zwischenabschnitt im Bereich der Seitenflächen plastisch verformt wird. Derartige seitliche Verformungen können einer Dauerfestigkeit des Zwischenabschnitts eher nachteilig entgegenwirken. Je nach Größe beziehungsweise Dicke des Zwischenabschnitts und der Seitenflächen können durch die zum Einsatz kommenden Strahlverfahren Einschlagkrater oder Beschusskrater in einer Größenordnung einer Wanddicke des Zwischenabschnitts auftreten. Daher können Verformungen der Seitenflächen in Form von Ecken oder Kerben gebildet werden, die dann eine Dauerfestigkeit des Zwischenabschnitts eher verringern.

Gemäß einer weiteren bevorzugten Ausführungsform der Erfindung kann vorgesehen sein, dass die äußere Oberfläche Außenflächen des ersten Befestigungsabschnitts, des zweiten Befestigungsabschnitts und des Zwischenabschnitts umfasst und dass die Außenflächen des ersten Befestigungsabschnitts und/oder des zweiten Befestigungsabschnitts und/oder des Zwischenabschnitts bearbeitet sind. Es ist also insbesondere möglich, wahlweise einen oder beide Befestigungsabschnitte und/oder den Zwischenabschnitt mit einem Strahlverfahren Oberflächen zu behandeln. Alternativ zum Kugelstrahlen, das auch als "Shot-Peening"-Verfahren bekannt ist, kann durch das sogenannte "Laser-Peening"-Verfahren eine Dicke der Druckeigenspannungsschicht noch weiter vergrößert werden, und zwar um etwa einen Millimeter.

Günstig ist es, wenn das erfindungsgemäße Wirbelsäulenstabilisierungssystem mindestens eines der oben beschriebenen Verbindungselemente umfasst. Es weist dann ebenfalls die oben im Zusammenhang mit den bevorzugten Ausführungsformen der Verbindungselemente beschriebenen Vorteile auf.

Verbindungselemente lassen sich auf einfache und sichere Weise an einem Knochen festlegen, wenn die mindestens eine erste und/oder die mindestens eine zweite Knochenbefestigungseinrichtung in Form einer Knochenschraube ausgebildet sind.

Um das Wirbelsäulenstabilisierungssystem möglichst optimal an individuelle Physiologien von Patienten anpassen zu können, ist es günstig, wenn die Knochenbefestigungseinrichtung einen Knochenverankerungsabschnitt und einen in einer Justagestellung gelenkig an diesem gelagerten Halteabschnitt umfasst, welcher Halteabschnitt eine Befestigungsabschnittsaufnahme für einen der Befestigungsabschnitte des Verbindungselements aufweist. Diese Ausgestaltung ermöglicht es, den Halteabschnitt in gewünschter Weise auszurichten, und zwar im Wesentlichen unabhängig von einer Orientierung des Knochenverankerungsabschnitts. Vorzugsweise ist der Halteabschnitt am Knochenverankerungsabschnitt in der Justagestellung polyaxial verschwenkbar gehalten und in einer Implantationsstellung unbeweglich festlegbar. Günstig kann es ferner sein, wenn mindestens einer der zwei Befestigungsabschnitte des Verbindungselementes eine Länge aufweist, die es ermöglicht, an zwei Knochenbefestigungseinrichtungen festgelegt zu werden. Werden letztere an benachbarten Wirbeln festgelegt, kann so ein erster Befestigungsabschnitt des Verbindungselements genutzt werden, um zwei benachbarte Wirbel starr miteinander zu verbinden, also einen Abschnitt der Wirbelsäule zu versteifen. Durch das besonders gestaltete Zwischenelement kann einer der beiden Wirbel zumindest teilweise beweglich mit einem weiteren Wirbel, an dem eine weitere Knochenbefestigungseinrichtung festgelegt ist, an welchem der zweite Befestigungsabschnitt festgelegt werden kann, gekoppelt werden, um eine Restbeweglichkeit in definierter Weise zu belassen.

Die nachfolgende Beschreibung bevorzugter Ausführungsformen der Erfindung dient im Zusammenhang mit der Zeichnung der näheren Erläuterung. Es zeigen:
- Figur 1:: eine schematische Gesamtansicht eines an einer Wirbelsäule festgelegten Wirbelsäulenstabilisierungssystems;
- Figur 2:: ein in Figur 1 dargestelltes Verbindungselement beim Montieren von Bewegungsbegrenzungsgliedern;

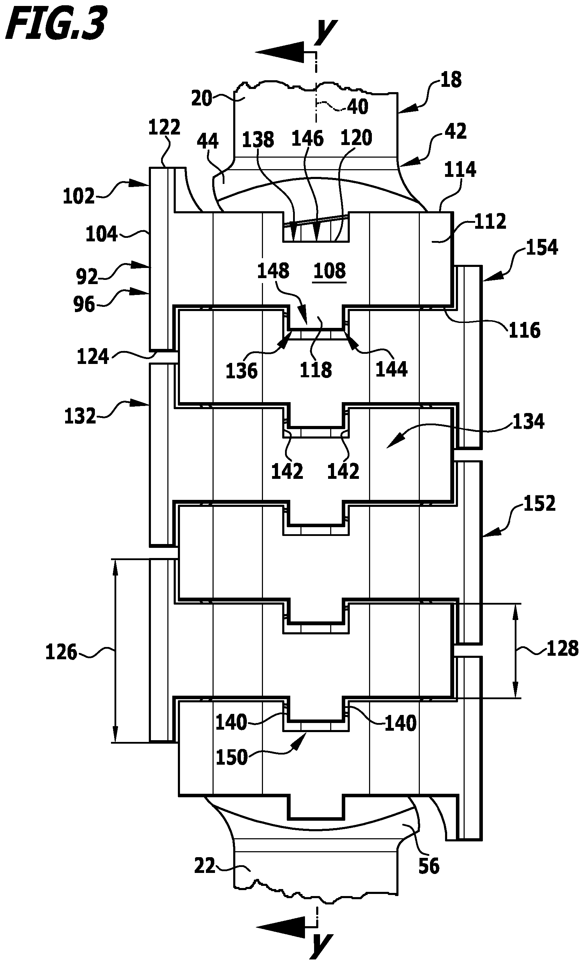

- Figur 3:: eine vergrößerte Seitenansicht eines Zwischenabschnitts des Verbindungselements aus Figur 1;
- Figur 4:: eine Schnittansicht längs Linie x-x in Figur 2;
- Figur 5:: eine Ansicht analog Figur 4 mit einer alternativen Ausführungsform von Bewegungsbegrenzungsgliedern;
- Figur 6:: eine Schnittansicht längs Linie y-y in Figur 3;
- Figur 7:: eine Schnittansicht analog Figur 4 bei gebogenem Zwischenabschnitt;
- Figur 8:: eine Seitenansicht eines seitlich gespannten Zwischenabschnitts;
- Figur 9:: eine Schnittansicht analog Figur 6 bei seitlicher Vorspannung wie in Figur 8 dargestellt;
- Figur 10:: eine perspektivische Ansicht eines Verbindungselements mit komplett durch ein Strahlverfahren bearbeiteter Oberfläche;
- Figur 11:: eine perspektivische Ansicht eines Verbindungselements, bei welchem lediglich der Zwischenabschnitt durch ein Strahlverfahren bearbeitet ist;
- Figur 12:: eine perspektivische Ansicht eines Verbindungselements, bei welchem bis auf die Seitenflächen des Zwischenabschnitts eine äußere Oberfläche vollständig durch ein Strahlverfahren bearbeitet ist;
- Figur 13:: eine perspektivische Ansicht eines Verbindungselements, bei welchem lediglich die Befestigungsabschnitte, nicht jedoch der Zwischenabschnitt, durch ein Strahlverfahren Oberflächen bearbeitet sind; und
- Figur 14:: eine vergrößerte schematische Ansicht des Bereichs A aus Figur 10.

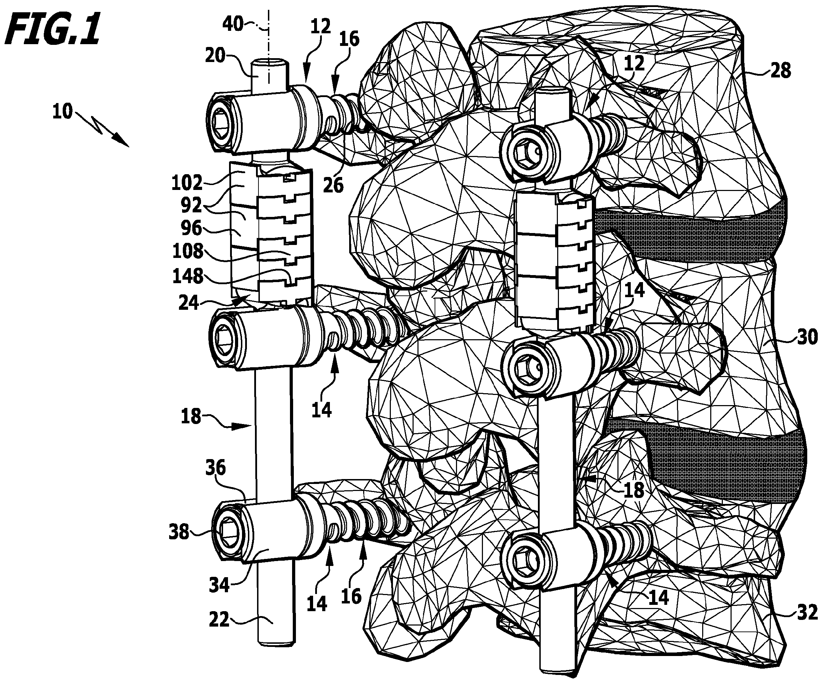

In Figur 1 ist schematisch ein insgesamt mit dem Bezugszeichen 10 bezeichnetes Wirbelsäulenstabilisierungssystem dargestellt. Es umfasst erste Knochenbefestigungseinrichtungen 12 und zweite Knochenbefestigungseinrichtungen 14, die bei dem in Figur 1 schematisch dargestellten Ausführungsbeispiel alle in Form identischer Knochenschrauben 16 ausgebildet sind, jedoch auch unterschiedlich ausgebildet sein können. Ferner umfasst das Wirbelsäulenstabilisierungssystem 10 im Wesentlichen stabförmige Verbindungselemente 18, welche einen ersten Befestigungsabschnitt 20 zum Festlegen an einer Knochenschraube 16, einen zweiten Befestigungsabschnitt 22 zum Festlegen an zwei Knochenschrauben 16 und einen zwischen dem ersten und dem zweiten Befestigungsabschnitt 20, 22 angeordneten oder ausgebildeten, mindestens teilweise flexiblen Zwischenabschnitt 24 umfassen.

Die Knochenschrauben 16 umfassen jeweils einen Knochenverankerungsabschnitt 26 mit einem Knochengewinde zum Verankern in einem Knochen, beispielsweise in einem der in Figur 1 schematisch dargestellten Wirbel 28, 30 und 32 einer Wirbelsäule. Des Weiteren umfasst jede Knochenschraube 16 einen in einer Justagestellung gelenkig am Knochenverankerungsabschnitt 26 gelagerten Halteabschnitt 34 in Form eines Gabelkopfes, welcher eine Befestigungsabschnittsaufnahme 36 für einen der Befestigungsabschnitte 20, 22 eines Verbindungselements 18 umfasst. Mittels einer Klemmschraube 38 kann ein in die Befestigungsabschnittsaufnahme 36 eingeführter Befestigungsabschnitt 20, 22 in einer Implantationsstellung klemmend fixiert werden. Durch Festlegen des Befestigungsabschnitts 20, 22 am Halteabschnitt 34 wird vorzugsweise auch eine Relativstellung zwischen dem Knochenverankerungsabschnitt 26 und dem Halteabschnitt 34 dauerhaft festgelegt.

Die Befestigungsabschnitte 20, 22 sind jeweils rundstabförmig ausgebildet und weisen somit einen kreisförmigen Querschnitt auf. Sie sind einstückig mit dem Zwischenabschnitt 24 ausgebildet. Vorzugsweise, wie bei dem in den Figuren dargestellten Ausführungsbeispiel eines Verbindungselements 18, definieren die Befestigungsabschnitte 20 und 22 eine gemeinsame Längsachse 40, die auch eine Längsachse des Zwischenabschnitts 24 definiert.

Der Zwischenabschnitt 24 ist an ein erstes Ende des Befestigungsabschnitts 20 angeformt und bildet im Übergangsbereich 42 zum Befestigungsabschnitt 20 eine im Wesentlichen flache, quaderförmige Endplatte 44 aus. An einer Längsseite derselben schließt sich quer ab von der Längsachse 40 ein gekrümmter Abschnitt 46 an. Dieser erstreckt sich über einen Winkelbereich von etwas mehr als 180°. An den gekrümmten Abschnitt 46 schließt sich ein flacher ebener Abschnitt 48 an, der wiederum in einen gekrümmten Abschnitt 50 übergeht, welcher ebenfalls von der Längsachse 40 weg weisend konvex gekrümmt ist. Die gekrümmten Abschnitte 46 und 50 weisen jedoch in zueinander entgegengesetzte Richtungen. Eine in Richtung auf den zweiten Befestigungsabschnitt 22 hin weisende Endfläche 52 der Endplatte 44 ist etwas gegen den ebenen Abschnitt 48 geneigt. An den gekrümmten Abschnitt 50 schließt sich wiederum ein ebener Abschnitt 54 an, welcher parallel zur Endfläche 52 verläuft. Die schlangenförmige Kontur des Zwischenabschnitts 24 setzt sich fort mit einem weiteren ebenen Abschnitt 48, einem sich daran anschließenden gekrümmten Abschnitt 50, einem sich daran anschließenden ebenen Abschnitt 54, einem sich daran anschließenden gekrümmten Abschnitt 46, einem sich daran anschließenden ebenen Abschnitt 48 und einem letzten, sich daran anschließenden gekrümmten Abschnitt 50, welcher in eine zur Endplatte 44 entsprechend geformte Endplatte 56 übergeht, welche eine in Richtung des ersten Befestigungsabschnitts 20 weisende Endfläche 58 aufweist, die parallel zu den ebenen Abschnitten 54 und damit auch zur Endfläche 52 verläuft.

Eine Steifigkeit des Zwischenabschnitts 24 liegt je nach Wahl des Materials, aus welchem das Verbindungselement 18 hergestellt ist, in einem Bereich von etwa 30 N/mm bis etwa 150 N/mm. Der Zwischenabschnitt 24 ist aus einem insgesamt im Wesentlichen flachen Material gebildet beziehungsweise aus einem Vollmaterial durch spanabhebende Bearbeitung, beispielsweise Fräsen, oder Erodieren geformt. Quer ab von der Längsachse 40 weist der Zwischenabschnitt 24 zwei in einer Grundstellung, in welcher auf den Zwischenabschnitt 24 keine äußeren Kräfte einwirken, parallel zueinander und voneinander weg weisende Seitenflächen 60 und 62 auf. Diese verlaufen zudem parallel zu einer die Längsachse 40 enthaltenden Symmetrieebene 64 des Verbindungselements 18.

Durch die schlangenförmige Ausgestaltung des Zwischenabschnitts 24 sind bei dem in den Figuren dargestellten Ausführungsbeispiel insgesamt sechs Ausnehmungen 66, 68 ausgebildet, wobei die Ausnehmungen 66 in dieselbe Richtung weisen wie die, in welche die konvex gekrümmten Abschnitte 50 weisend geöffnet sind, die Ausnehmungen 68 jedoch in die entgegengesetzte Richtung, also in die Richtung, in die die konvex gekrümmten Abschnitte 46 weisen. Jede der Ausnehmungen 66, 68 definiert eine Einführöffnung 70 beziehungsweise 72, die jeweils einem gekrümmten Abschnitt 46 beziehungsweise 50 gegenüberliegt und in die jeweils entgegengesetzte Richtung weist. Jede Ausnehmung 66, 68 wird durch zwei in Richtung der jeweiligen Einführöffnung 70, 72 aufeinander zu laufende ebene Abschnitte 48 und 54 begrenzt, so dass sich ein Querschnitt der jeweiligen, in etwa tropfenförmigen Ausnehmung 66, 68 von der Einführöffnung 70 beziehungsweise 72 in Richtung auf den die Ausnehmungen 66, 68 weiter begrenzenden gekrümmten Abschnitte 46, 50 hin zunimmt. Damit definiert jede Einführöffnung 70, 72 eine Engstelle 74. Die Ausnehmungen 66, 68 sind somit jeweils in einer Richtung quer zur Längsachse 40 seitlich geöffnet.

Eine Dicke 76 des Zwischenabschnitts 24 im Bereich der gekrümmten Abschnitte 46, 50 ist größer als im Bereich der ebenen Abschnitte 48, 54. Die Dicke 76 beträgt etwa das 1,1fache bis etwa 1,5fache der Dicke 78, vorzugsweise etwa das 1,3fache bis etwa 1,35fache. Bei dem in den Figuren dargestellten Ausführungsbeispiel beträgt die Dicke 76 etwa 0,8 mm, die Dicke 78 etwa 0,6 mm. Ein Innenradius der gekrümmten Abschnitte 46, 50 beträgt etwa 1,6 mm, ein Außenradius etwa 2,2 mm.

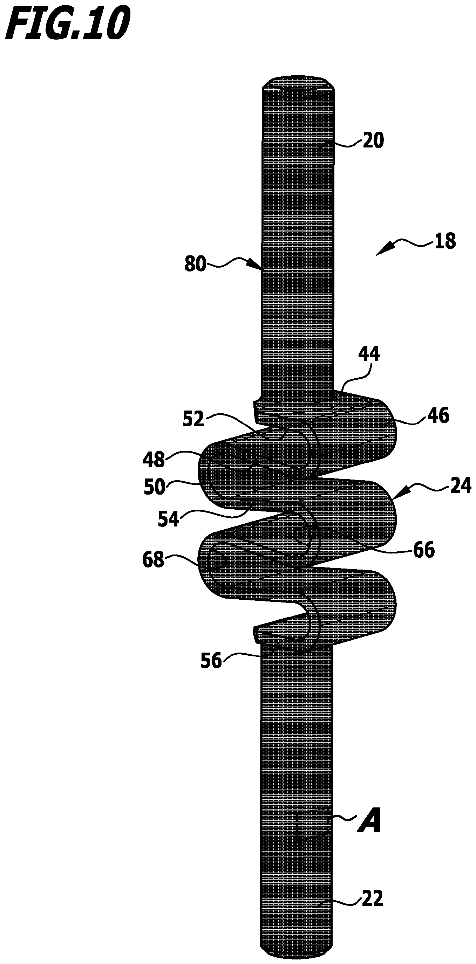

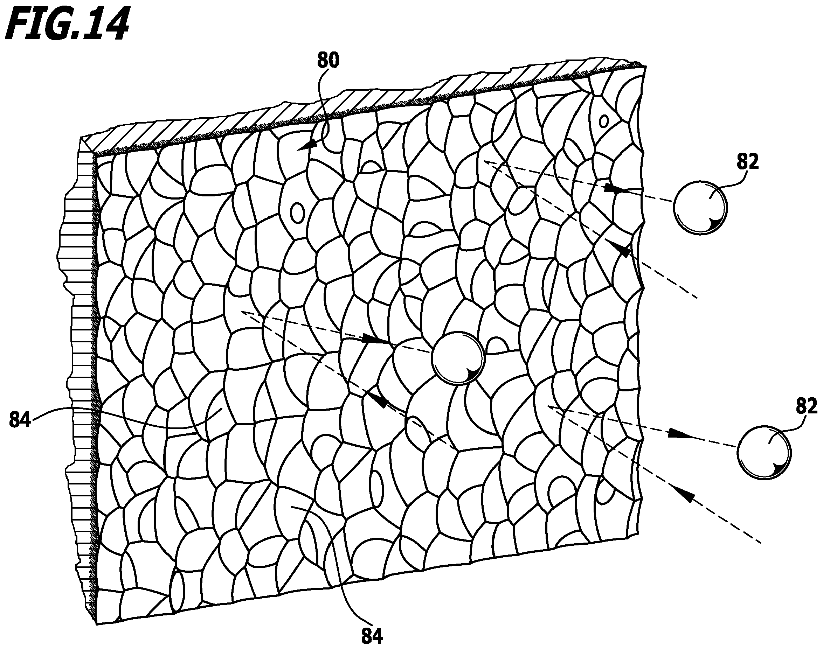

Zur Erhöhung der Dauerfestigkeit des Verbindungselements 18 kann dessen äußere Oberfläche 80 durch ein Strahlverfahren mindestens teilweise bearbeitet sein. Dies ist schematisch in den Figuren 10 bis 14 dargestellt.

In Figur 10 ist ein Verbindungselement 18 schematisch dargestellt, dessen äußere Oberfläche 80 komplett gestrahlt ist. Als Strahlverfahren können zum Beispiel Kugelstrahlverfahren mit Kugeln 82 zum Einsatz kommen, wobei die Kugeln 82 aus Glas oder Stahl hergestellt sind. Das Kugelstrahlen ist ein Kaltbearbeitungsverfahren, bei dem die Oberfläche 80 des Verbindungselements 18 mit Kugeln 82 bestrahlt wird, wobei jede Kugel 82, die auf die Oberfläche 80 trifft, dabei wie ein winziger Schmiedehammer wirkt, der eine flache Kalotte 84 auf der Oberfläche hinterlässt. Damit eine Kalotte 84 entstehen kann, muss eine Oberflächenschicht des Verbindungselements 18 gedehnt werden. Tiefer liegende Werkstoffschichten dagegen versuchen die Oberfläche 80 wieder in den Ursprungszustand zurückzubringen. Dabei wird unter den Kalotten 84 jeweils ein Bereich kaltverformten Werkstoffes mit einer hohen Druckeigenspannung erzeugt. Überlappende Kalotten 84 ergeben eine gleichmäßig hohe Druckeigenspannung in einer Randzone des Verbindungselements 18.

Innerhalb einer Druckeigenspannungsschicht können keine Risse entstehen und sich dort auch nicht ausbreiten. Da nahezu alle Ermüdungs- und Spannungsrisskorrosionsschäden normalerweise von der Oberfläche 80 ausgehen, wird eine Lebensdauer des Verbindungselements 18 durch das Kugelstrahlen beträchtlich erhöht. Die durch das Kugelstrahlen erzeugten Druckeigenspannungen in der Oberflächenschicht betragen etwa die Hälfte der Werkstoffzugfestigkeit. Bei vielen Werkstoffen erhöht sich zudem eine Oberflächenhärte aufgrund der Kaltverfestigung durch das Kugelstrahlen.

Alternativ zum Kugelstrahlen, das auch als "Shot-Peening"-Verfahren bezeichnet wird, kann auch das sogenannte "Laser-Peening"-Verfahren zum Einsatz kommen. Es beruht auf durch gepulste Laserbestrahlung generierte Schockwellen zur Erzeugung von Druckeigenspannungen. Durch das "Laser-Peening"-Verfahren kann die Druckeigenspannungsschicht weiter vergrößert werden, beispielsweise um etwa einen Millimeter.

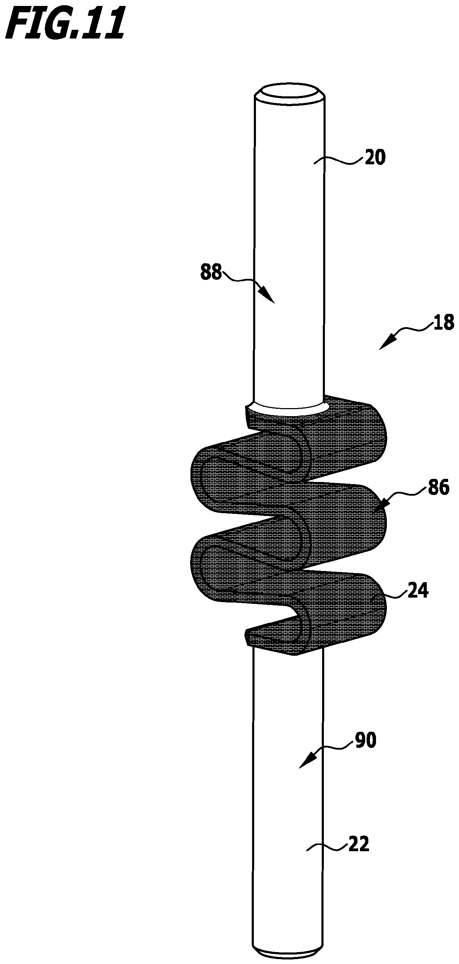

Alternativ zur Strahlbehandlung der gesamten äußeren Oberfläche 80 kann, wie bei dem in Figur 11 schematisch dargestellten Verbindungselement 18 nur eine Außenfläche 86 des Zwischenabschnitts 24 strahlbehandelt werden. Außenflächen 88 und 90 der Befestigungsabschnitt 20 und 22 werden bei der Strahlbehandlung des Zwischenabschnitts 24 abgedeckt und bleiben damit nicht gestrahlt.

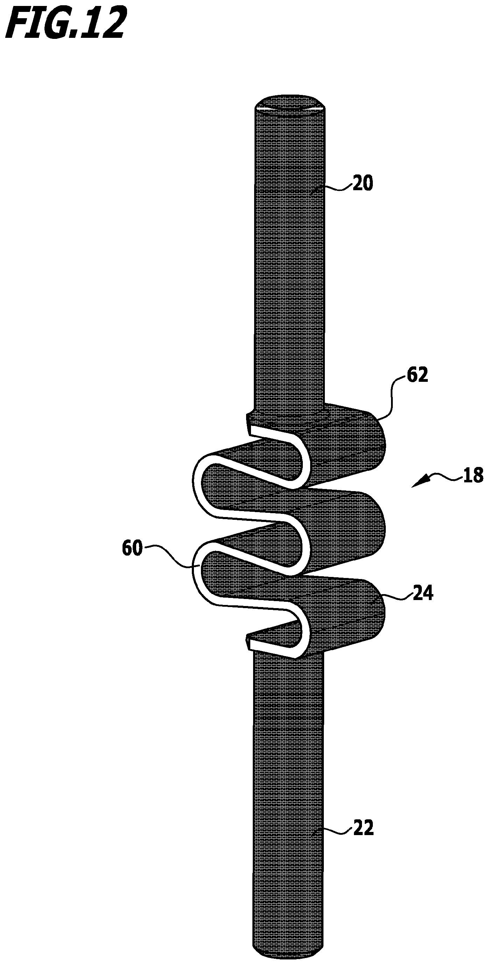

Die durch die Außenflächen 86, 88 und 90 definierte äußere Oberfläche 80 kann, wie bei dem in Figur 12 schematisch dargestellten Ausführungsbeispiel eines Verbindungselements 18, auch bis auf die Seitenflächen 60 und 62 vollständig gestrahlt sein. Diese werden bei der Strahlbehandlung abgedeckt, so dass sie nicht gestrahlt verbleiben. Damit wird verhindert, dass der dünnwandige Zwischenabschnitt 24 an den Seitenflächen 60, 62 plastisch verformt wird. Durch das Kugelstrahlen bedingte seitliche Verformungen können im ungünstigsten Fall hinsichtlich der durch das Strahlen bewirkten Dauerfestigkeit sogar nachteilig wirken. In Abhängigkeit einer Größe der Kugeln 82 können die entstehenden Kalotten 84 insbesondere in einer Größenordnung der Dicken 76 und 78 liegen.

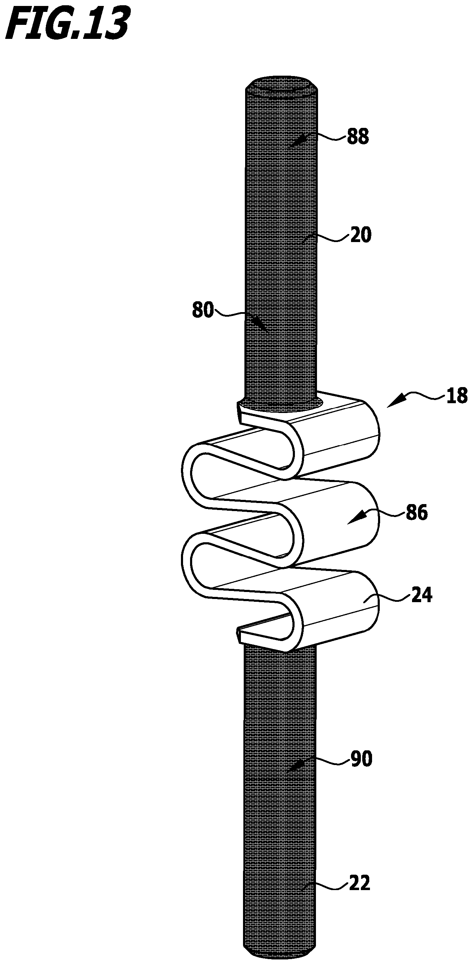

Eine weitere Variante einer teilweise gestrahlten äußeren Oberfläche 80 eines Verbindungselements 18 ist schematisch in Figur 13 dargestellt. Lediglich die Außenflächen 88 und 90 sind dabei strahlbehandelt. Der Zwischenabschnitt 24 bleibt ungestrahlt, da er bei der Strahlbehandlung komplett abgedeckt wird.

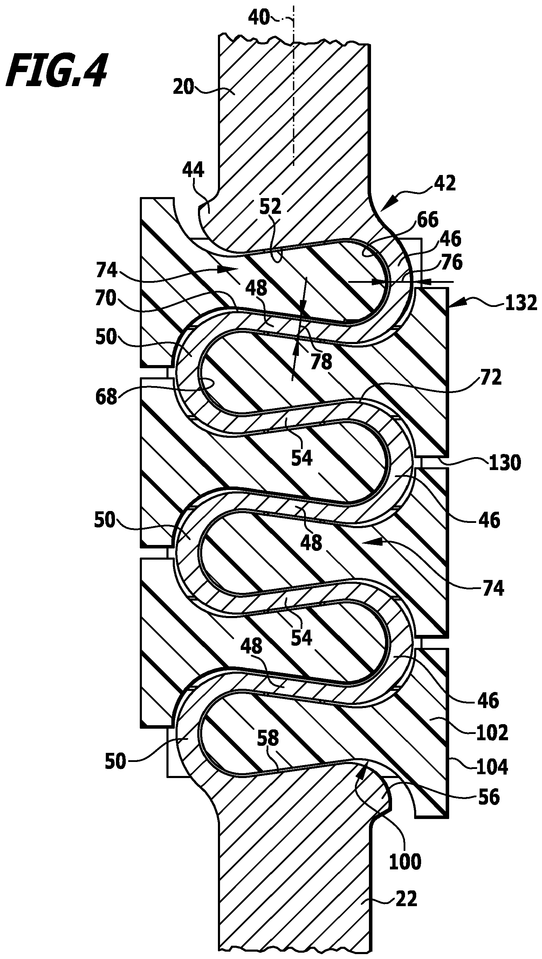

Um die mechanischen Eigenschaften, insbesondere eine Steifigkeit des Zwischenabschnitts 24 des Verbindungselements 18 zu beeinflussen, sind Bewegungsbegrenzungsglieder 92 entsprechend der Zahl der durch den Zwischenabschnitt 24 definierten Ausnehmungen 66, 68 vorgesehen. Jedes Bewegungsbegrenzungsglied 92 umfasst einen Füllkörper 94, welcher im Schnitt, wie in Figur 4 schematisch dargestellt, im Wesentlichen eine Tropfenform aufweist und die Ausnehmungen 66, 68 mit Spiel behaftet nahezu ausfüllt.

Des Weiteren umfasst jedes Bewegungsbegrenzungsglied 92 eine Anschlageinrichtung 96, welche parallel oder im Wesentlichen parallel zur Längsachse 40 und, bei alternativen Ausführungsformen auch wahlweise, in Umfangsrichtung bezogen auf die Längsachse 40 wirkt. Der Füllkörper 94 greift somit in einer Grundstellung des Verbindungselements 18, in welcher auf den Zwischenabschnitt 24 keine äußeren Kräfte wirken, beispielsweise Kräfte, die über die Befestigungsabschnitte 20, 22 eingeleitet werden, mindestens teilweise in eine Ausnehmung 66, 68 ein. Eine Breite des Füllkörpers 94 ist etwas größer als ein Abstand 98 zwischen den Seitenflächen 60 und 62.

Am Füllkörper 94 ist ferner eine Querschnittsverjüngung 100 verwirklicht, die auch als Einschnürung bezeichnet werden kann. Wie in Figur 4 schematisch dargestellt, befindet sich diese beim in eine Ausnehmung 66, 68 eingeführten Füllkörper 94 im Bereich der Einführöffnung 70 beziehungsweise 72.

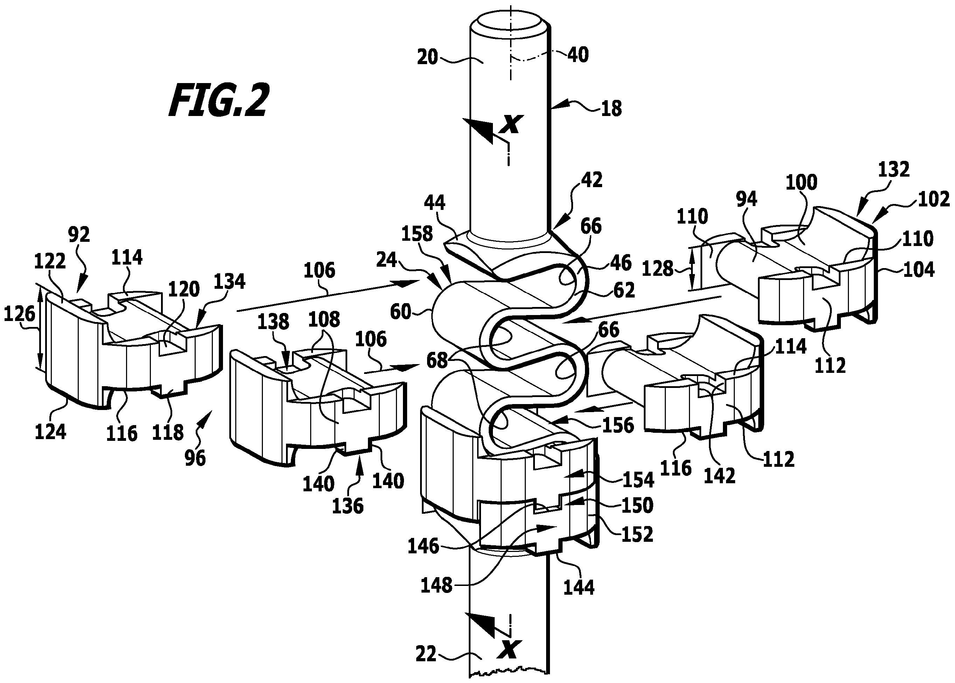

Ausgehend von der Querschnittsverjüngung 100 verbreitert sich der Füllkörper 94 wieder und geht in ein im Wesentlichen blättchenförmig ausgebildetes Schließelement 102 über, mit welchem die Einführöffnung 70, 72 praktisch vollständig verschlossen werden kann. Eine von der Längsachse 40 weg weisende Außenfläche 104 des Schließelements 102 ist konvex gekrümmt und bildet einen Ausschnitt einer Zylinderoberfläche. Die Querschnittsverjüngung 100 am Bewegungsbegrenzungsglied 92 sowie die an den Ausnehmungen 66, 68 vorgesehenen, im Querschnitt verringerten Einführöffnungen 70 und 72 verhindern, dass die in einer durch Pfeile 106 in Figur 2 repräsentierten Einführrichtung in die Ausnehmungen 66 und 68 eingeführten Füllkörper 94 wieder aus diesen entnehmbar oder verlierbar sind, ohne die Einführöffnungen 70, 72 aufzuweiten, beispielsweise durch Aufdehnen der Ausnehmungen 66 und 68 durch wechselseitiges Verschwenken von Längsachsen der Befestigungsabschnitte 20 und 22 relativ zueinander. Alternativ kann eine Aufweitung der Einführöffnungen 70 und 72 auch durch Ausübung einer Zugkraft auf die Befestigungsabschnitte 20 und 22 in einer Richtung voneinander weg erreicht werden.

Jedes Bewegungsbegrenzungsglied 92 ist symmetrisch zur Symmetrieebene 64 ausgebildet und umfasst zusätzlich zum Schließelement 102 mindestens ein, bei den in den Figuren dargestellten Ausführungsbeispielen zwei seitliche Verschlusselemente 108 zum seitlichen Verschließen der Ausnehmungen 66, 68. Die Verschlusselemente 108 weisen ebene Anschlagflächen 110 auf, die in Richtung auf die Längsachse 40 hin weisen und parallel zueinander verlaufen. Von der Längsachse 40 weg weisende Außenflächen 112 der Verschlusselemente 108 sind konvex gekrümmt und bilden einen Teil einer Zylinderoberfläche. Insbesondere setzen sie die direkt angrenzende Außenfläche 104 des Schließelements 102 fort.

Jedes Verschlusselement 108 weist zwei voneinander weg weisende Anschlagflächen 114 und 116 auf, die senkrecht zur Längsachse 40 verlaufende Ebenen definieren. Von der Anschlagfläche 116 steht ein kleiner quaderförmiger Vorsprung 118 ab. Des Weiteren ist an jedem Verschlusselement 108 eine Ausnehmung 120 vorgesehen, die sich etwas in Richtung auf die Längsachse 40 hin in den Füllkörper 94 hinein erstreckt und in einer Richtung entgegengesetzt zum Vorsprung 118 weist.

Die Bewegungsbegrenzungsglieder 92 werden, wie schematisch in Figur 2 dargestellt, mit den Füllkörpern 94 alternierend in die Ausnehmungen 66, 68 eingeschoben. Die Vorsprünge 118 sowie die Ausnehmungen 120 sind so angeordnet und geformt, dass ein Vorsprung 118 in eine Ausnehmung 120 eines benachbarten Bewegungsbegrenzungsglieds 92 im Wesentlichen formschlüssig eingreift. Dabei liegt jeweils eine Anschlagfläche 114 eines Bewegungsbegrenzungsglieds 92 im Wesentlichen flächig an einer Anschlagfläche 116 des benachbarten Bewegungsbegrenzungsglieds 92 an.

Die Verschlusselemente 108 verschließen die Ausnehmungen 66, 68 auf zwei einander entgegengesetzten Seiten. Zusammen mit dem Schließelement 102 ergibt sich somit eine im Wesentlichen U-förmige Anordnung der Verschlusselemente 108 sowie des Schließelements 102, um einen direkten Zugang zu den Ausnehmungen 66, 68 zu verschließen und den Zwischenabschnitt 24 zu umgreifen.

Jedes Schließelement 102 weist zwei parallel zueinander verlaufende Anschlagflächen 122 und 124 auf, die senkrecht zur Längsachse 40 verlaufende Ebenen definieren und parallel zu den Anschlagflächen 114 und 116 verlaufen. Ein Abstand 126 zwischen den Anschlagflächen 122 und 124 ist nur wenig kleiner als das Doppelte eines Abstands 128 zwischen den Anschlagflächen 114 und 116. Durch die alternierende Anordnung benachbarter Bewegungsbegrenzungsglieder 92 liegen sich, wie insbesondere in den Figuren 4, 5 und 7 gut zu erkennen, Anschlagflächen 122 und 124 nicht direkt benachbart angeordneter Bewegungsbegrenzungsglieder 92, sondern übernächster Nachbarn von Bewegungsbegrenzungsgliedern 92, einander gegenüber. Zwischen Ihnen wird jeweils ein Spalt 130 definiert.

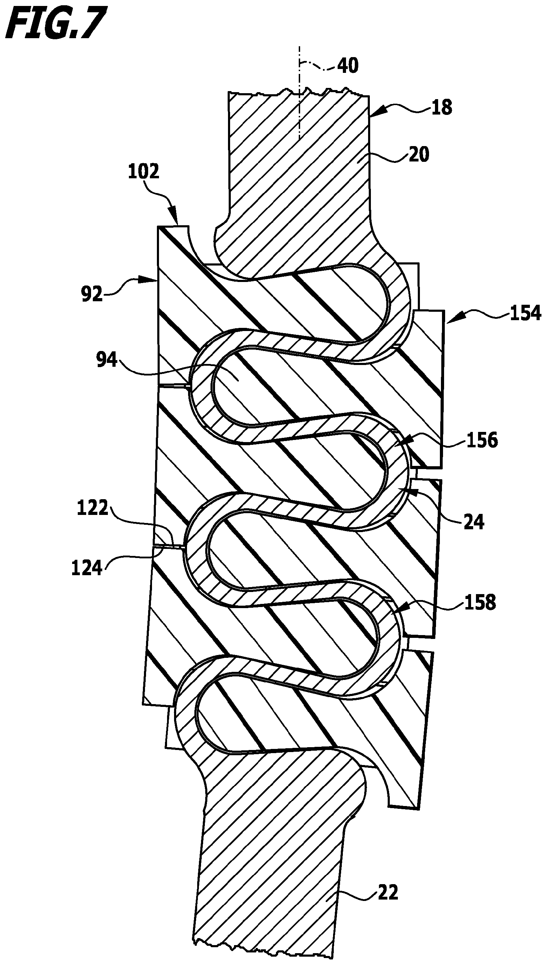

Sowohl das Schließelement 102 als auch die Verschlusselemente 108 bilden Anschläge 132 beziehungsweise 134 aus, die dazu dienen, eine Bewegung des Zwischenabschnitts 24 zu limitieren. Eine Verbiegung des Zwischenabschnitts 24 ist, wie in Figur 7 schematisch dargestellt, nur in dem Umfang möglich, bis die Anschlagflächen 122 und 124 aneinander anstoßen. Die Anschlagflächen 114 und 116 der Anschläge 134 begrenzen insbesondere eine Kompression des Zwischenabschnitts 24.

Die Vorsprünge 118 bilden, ebenso wie die Ausnehmungen 120, weitere Anschläge 136 und 138 aus. In Umfangsrichtung weisende Anschlagflächen 140 der Anschläge 136 sowie in Umfangsrichtung weisende Anschlagflächen 142 der Anschläge 138 wirken bezogen auf die Längsachse 40 in Umfangsrichtung zusammen und verhindern eine Rotation benachbarter Bewegungsbegrenzungsglieder 92 um die Längsachse 40.

Ferner bilden die Vorsprünge 118 und die Ausnehmungen 120 Kupplungsglieder 144 und 146 von insgesamt mit dem Bezugszeichen 148 bezeichneten Kupplungseinrichtungen, welche wiederum selbst einen Teil einer insgesamt mit dem Bezugszeichen 150 bezeichneten Antiverwindungseinrichtung bilden, die dazu dient, im Wesentlichen eine Verwindung des Zwischenabschnitts 24 um die Längsachse 40 zu verhindern. Die Antiverwindungseinrichtung 150 umfasst also insbesondere die beiden in Umfangsrichtung wirkenden Anschläge 136 und 138. Durch die Kupplungseinrichtung 148 können benachbarte Bewegungsbegrenzungsglieder 92, deren Füllkörper 94 in benachbarte Ausnehmungen 66, 68 des Zwischenabschnitts 24 eingreifen, miteinander gekoppelt werden. Die Kupplungsglieder 144 und 146 stehen bei den in den Figuren dargestellten Ausführungsbeispielen im Wesentlichen formschlüssig miteinander in Eingriff. Insgesamt umfasst jedes der beschriebenen Bewegungsbegrenzungsglieder 92 zwei unterschiedliche Kupplungsglieder 144 und 146, die jeweils einer Kupplungseinrichtung 148 zugeordnet sind. Die Kupplungsglieder 144 und 146 sind zudem in entgegengesetzte Richtungen weisend ausgebildet und parallel oder im Wesentlichen parallel zur Längsachse 40 ausgerichtet.

Durch die beschriebene Ausgestaltung definieren die mit dem Zwischenabschnitt 24 in Eingriff stehenden Bewegungsbegrenzungsglieder 92 eine gemeinsame und im Wesentlichen zylindrische Hüllkontur 152.

Die Bewegungsbegrenzungsglieder 92 sind alle identisch und einstückig ausgebildet. Insbesondere sind sie in Form eines Spritzgussteils ausgebildet, und zwar aus einem biokompatiblen Material. Bei diesem handelt es sich vorzugsweise um einen Kunststoff, insbesondere um Polyethylen, Polyetheretherketon, Polyurethan oder Polycarbonaturethan. Vorteilhaft sind ferner faserverstärkende Kunststoffe.

Das Verbindungselement 18 umfasst ferner eine Rückstelleinrichtung 154 zum automatischen Rückführen des Verbindungselements 18 aus einer aus der Grundstellung ausgelenkten Auslenkstellung in die Grundstellung zurück. Die Rückstelleinrichtung 154 umfasst ein Rückstellelement 156 zum automatischen Rückführen des Verbindungselements 18 aus der Auslenkstellung in die Grundstellung zurück. Es ist, wie bei den in den Figuren dargestellten Ausführungsbeispielen, in Form eines Federelements 158 ausgebildet. Das Federelement 158 wird definiert durch eine schlangenförmig gewundene Blattfeder, die den Zwischenabschnitt 24 bildet.

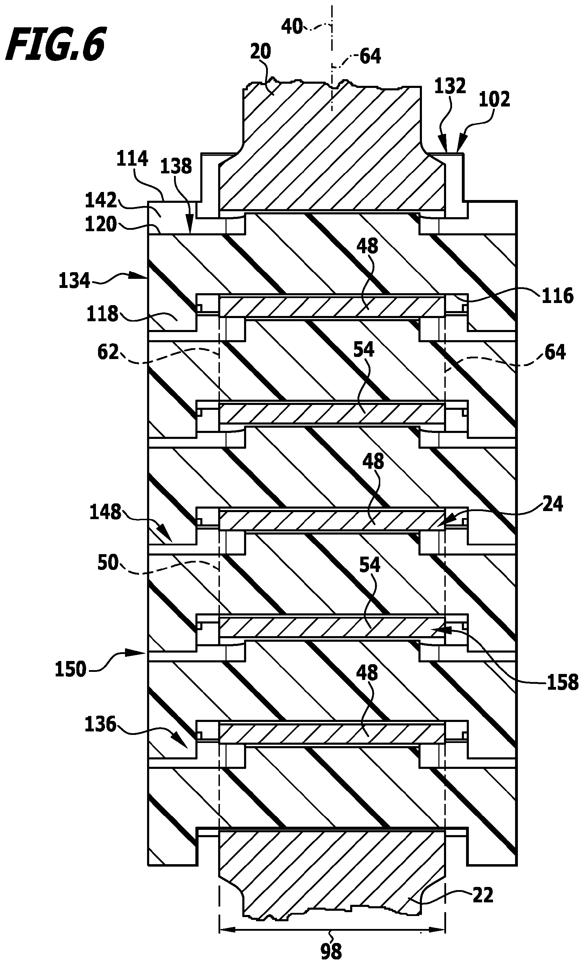

Wird das Verbindungselement 18 durch Verschwenken der Befestigungsabschnitte 20 und 22 zum Beispiel in einer in den Figuren 4 und 7 dargestellten Schnittebene relativ zueinander verbogen, so wird nach Entlasten der Befestigungsabschnitte 20, 22 das Verbindungselement wieder in die in den Figuren 4 und 6 dargestellte Grundstellung automatisch zurück überführt, und zwar durch die Rückstelleinrichtung 154.

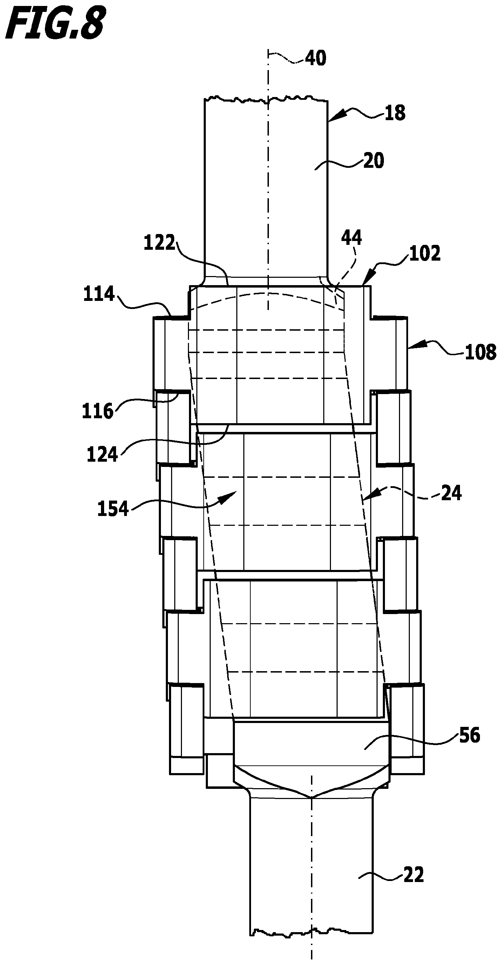

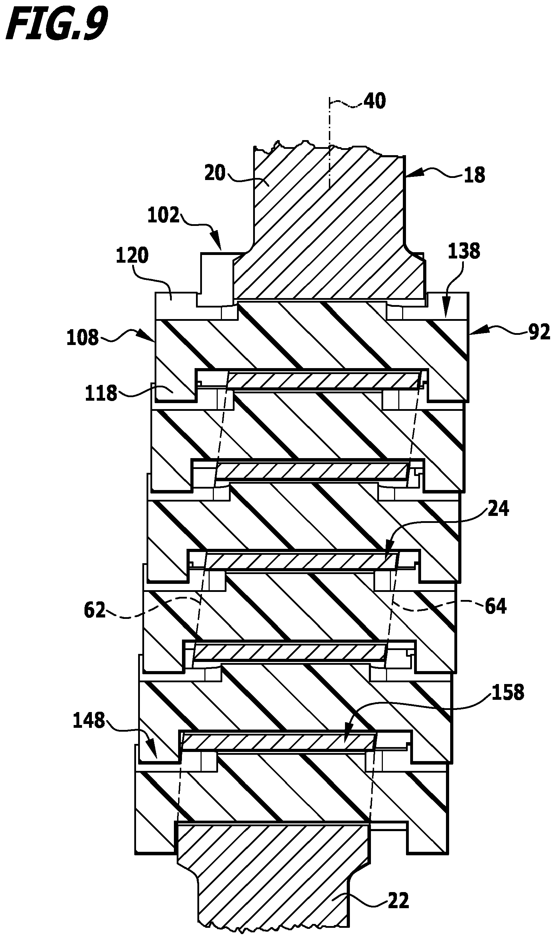

Eine seitliche Vorspannung der Befestigungsabschnitte 20, 22 gegeneinander in einer Ebene senkrecht zu einer in den Figuren 4 und 6 dargestellten Flexionsebene führt zu einer sukzessiven Auslenkung benachbarter Bewegungsbegrenzungsglieder 92 in einer Richtung senkrecht zur Einführrichtung 106, wie dies in den Figuren 8 und 9 schematisch dargestellt ist. Eine maximale Auslenkung wird hier wiederum begrenzt durch die Anschläge 136 und 138.

Durch eine Auswahl der Materialien, aus denen die Bewegungsbegrenzungsglieder 92 hergestellt werden sowie durch Auswahl der Materialien, aus denen das Verbindungselement 18 hergestellt wird, können Steifigkeiten des Verbindungselements 18 praktisch beliebig eingestellt werden. Ein maximaler Flexionswinkel zwischen den Befestigungsabschnitten 20, 22 kann definiert werden durch eine Höhe der Schließelemente 102, das heißt durch den Abstand 126. Über diesen wird gleichzeitig eine Breite des Spalts 130 vorgegeben. Je breiter der Spalt, umso größer eine mögliche Flexion des Zwischenabschnitts 24.

Die Bewegungsbegrenzungsglieder 92 können in der beschriebenen Weise das Wirbelsäulenstabilisierungssystem 10 stabilisieren. Sie dienen quasi als Extensionsstopper, indem sie eine Bewegung des Zwischenabschnitts 24 bei axialer Kompression einschränken. Je nach Spiel der Bewegungsbegrenzungsglieder 92 kann eine Kennlinie in Kompression beeinflusst werden. Bevorzugt kommt es dabei zu einem progressiven Kennlinienverlauf.

Dadurch, dass die Bewegungsbegrenzungsglieder 92 den Zwischenabschnitt 24 von außen umfassen und gegenseitig ineinander eingreifen, bilden sie quasi eine äußere Gliederkette um den Zwischenabschnitt 24. Die Wirkkräfte werden dabei durch die Verschlusselemente 108 aufgenommen. Eine Steifigkeit des Verbindungselements 18 in Scherung wird damit erhöht. Dies bedeutet gleichzeitig eine Erhöhung der Steifigkeit des Verbindungselements 18 in axialer Rotation.

Die Kupplungseinrichtungen 148 dienen zusätzlich dazu, die Bewegungsbegrenzungsglieder unverlierbar miteinander und am Zwischenabschnitt 24 zu verriegeln. Wie bereits dargelegt, wird beim Montieren der Bewegungsbegrenzungsglieder 92 vorzugsweise der Zwischenabschnitt etwas elastisch aufgebogen und die Bewegungsbegrenzungsglieder 92 beginnend beispielsweise vom Bewegungsabschnitt 20 aus nacheinander in die Ausnehmungen 66, 68 eingeschoben.

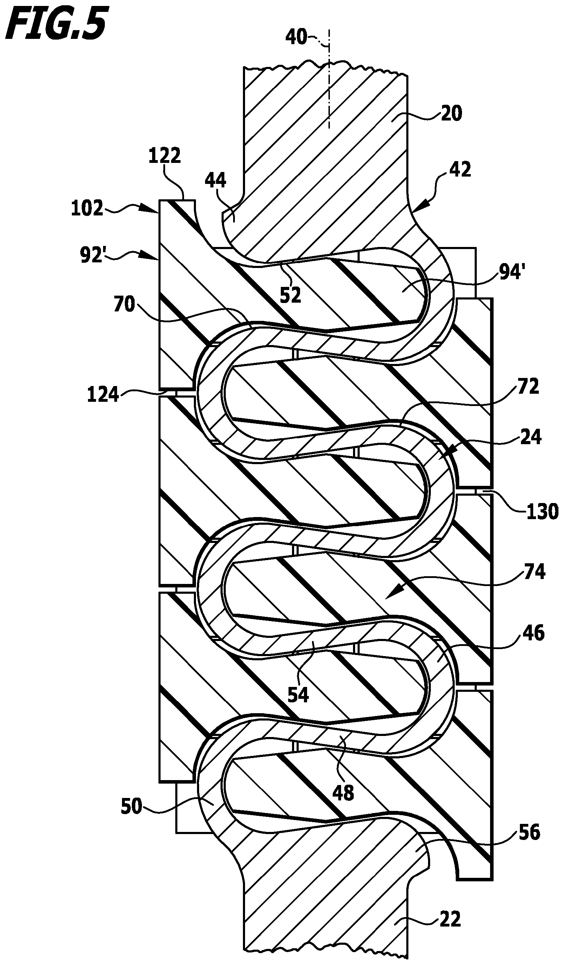

Eine Montage der Bewegungsbegrenzungsglieder 92 kann insbesondere etwas vereinfacht werden, wenn eine Querschnittsform der Füllkörper 94 verändert wird. Bei der in Figur 5 schematisch dargestellten Variante 92' eines Bewegungsbegrenzungsglieds ist ein vom Schließelement 102 weg weisendes Ende des Füllkörper 94' sich konisch verjüngend ausgebildet. Bei der Montage ist daher im Vergleich zum Bewegungsbegrenzungsglied 92 eine etwas geringfügigere Aufweitung der Einführöffnungen 70, 72 erforderlich.

## Patentansprüche

1. Verbindungselement (18) für ein Wirbelsäulenstabilisierungssystem (10) mit einem ersten Befestigungsabschnitt (20) zum Festlegen an einer ersten Knochenbefestigungseinrichtung (12), einem zweiten Befestigungsabschnitt (22) zum Festlegen an einer zweiten Knochenbefestigungseinrichtung (14) und einem zwischen dem ersten und dem zweiten Befestigungsabschnitt (20, 22) angeordneten oder ausgebildeten, mindestens teilweise flexiblen Zwischenabschnitt (24), welcher Zwischenabschnitt (24) mindestens eine in einer Richtung quer zu einer vom Zwischenabschnitt (24) definierten Längsachse (40) seitlich geöffnete Ausnehmung (66, 68) aufweist, wobei mindestens ein Bewegungsbegrenzungsglied (92) vorgesehen ist, welches einen Füllkörper (94) und mindestens eine parallel oder im Wesentlichen parallel zur Längsachse (40) wirkende Anschlageinrichtung (96) umfasst, und wobei der Füllkörper (94) in einer Grundstellung, in welcher auf den Zwischenabschnitt (24) keine äußeren Kräfte wirken, in die mindestens eine Ausnehmung (66, 68) mindestens teilweise eingreift, **dadurch gekennzeichnet, dass** das mindestens eine Bewegungsbegrenzungsglied (92) eine in Umfangsrichtung wirkende Anschlageinrichtung (96) umfasst.

2. Verbindungselement nach Anspruch 1, **dadurch gekennzeichnet, dass** die Anschlageinrichtung (96) mindestens einen am Füllkörper (94) angeordneten, ausgebildeten und/oder von diesem abstehenden Anschlag (132, 134, 136, 138) umfasst, welcher parallel oder im Wesentlichen parallel zur Längsachse (40) und in Umfangsrichtung wirkt.

3. Verbindungselement nach einem der voranstehenden Ansprüche, **dadurch gekennzeichnet, dass** mehrere Bewegungsbegrenzungsglieder (92) vorgesehen sind und dass Anschlageinrichtungen (96) benachbarter Bewegungsbegrenzungsglieder (92) in der Grundstellung mindestens teilweise kontaktlos einander gegenüberliegen und in einer aus der Grundstellung ausgelenkten Stellung aneinander anliegen.

4. Verbindungselement nach einem der voranstehenden Ansprüche, **dadurch gekennzeichnet, dass** jedes Bewegungsbegrenzungsglied (92) eine Anschlageinrichtung (96) mit zwei in entgegengesetzte Richtungen weisenden Anschlägen (136, 138) umfasst.

5. Verbindungselement nach einem der voranstehenden Ansprüche, **dadurch gekennzeichnet, dass** das mindestens eine Bewegungsbegrenzungsglied (92) und/oder der Zwischenabschnitt (24) spiegelsymmetrisch zu einer die Längsachse (40) enthaltenden Spiegelebene (64) ausgebildet sind.

6. Verbindungselement nach einem der voranstehenden Ansprüche, **gekennzeichnet durch** eine Antiverwindungseinrichtung (150) zum Verhindern einer Verwindung des Zwischenabschnitts (24) um die Längsachse (40).

7. Verbindungselement nach einem der voranstehenden Ansprüche, **dadurch gekennzeichnet, dass** die mindestens eine Ausnehmung (66, 68) in der Grundstellung eine quer zur Längsachse (40) und von dieser weg weisende Einführöffnung (70, 72) aufweist zum Einführen des Füllkörpers (94) in einer Richtung quer zur Längsachse (40) und auf diese hin.

8. Verbindungselement nach Anspruch 7, **dadurch gekennzeichnet, dass** das Bewegungsbegrenzungsglied (92) ein Schließelement (102) zum Verschließen der Einführöffnung (70, 72) umfasst.

9. Verbindungselement nach einem der voranstehenden Ansprüche, **dadurch gekennzeichnet, dass** das mindestens eine Bewegungsbegrenzungsglied (92) mindestens ein seitliches Verschlusselement (108) umfasst zum mindestens teilweisen seitlichen Verschließen der mindestens einen Ausnehmung (66, 68).

10. Verbindungselement nach Anspruch 9, **dadurch gekennzeichnet, dass** das mindestens eine seitliche Verschlusselement (108) seitlich vom Füllkörper (94) abstehend ausgebildet ist.

11. Verbindungselement nach einem der Ansprüche 9 und 10, **dadurch gekennzeichnet, dass** die Anschlageinrichtung (96) mindestens einen am Füllkörper (94) angeordneten, ausgebildeten und/oder von diesem abstehenden Anschlag (132, 134, 136, 138) umfasst, welcher parallel oder im Wesentlichen parallel zur Längsachse (40) und in Umfangsrichtung wirkt und dass das mindestens eine seitliche Verschlusselement (108) mindestens einen der parallel oder im Wesentlichen parallel zur Längsachse (40) oder in Umfangsrichtung wirkenden Anschläge (136, 138) umfasst oder trägt.

12. Verbindungselement nach einem der voranstehenden Ansprüche, **dadurch gekennzeichnet, dass** eine Steifigkeit des Zwischenabschnitts (24) einen Wert in einem Bereich von etwa 30 N/mm bis etwa 150 N/mm aufweist.

13. Verbindungselement nach einem der voranstehenden Ansprüche, **gekennzeichnet durch** eine mindestens teilweise durch ein Strahlverfahren bearbeitete äußere Oberfläche (80).

14. Wirbelsäulenstabilisierungssystem (10) umfassend mindestens eine erste Knochenbefestigungseinrichtung (12), mindestens eine zweite Knochenbefestigungseinrichtung (14) und ein Verbindungselement (18), welches Verbindungselement (18) einen ersten Befestigungsabschnitt (20) zum Festlegen an der mindestens einen ersten Knochenbefestigungseinrichtung (12), einen zweiten Befestigungsabschnitt (22) zum Festlegen an der mindestens einen zweiten Knochenbefestigungseinrichtung (14) und einen zwischen dem ersten und dem zweiten Befestigungsabschnitt (20, 22) angeordneten oder ausgebildeten, mindestens teilweise flexiblen Zwischenabschnitt (24) umfasst, welcher Zwischenabschnitt (24) mindestens eine quer zu einer vom Zwischenabschnitt (24) definierten Längsachse (40) seitlich geöffnete Ausnehmung (66, 68) aufweist, wobei mindestens ein Bewegungsbegrenzungsglied (92) vorgesehen ist, welches einen Füllkörper (94) und mindestens eine parallel oder im Wesentlichen parallel zur Längsachse (40) wirkende Anschlageinrichtung (96) umfasst, und wobei der Füllkörper (94) in einer Grundstellung, in welcher auf den Zwischenabschnitt (24) keine äußeren Kräfte wirken, in die mindestens eine Ausnehmung (66, 68) mindestens teilweise eingreift, **dadurch gekennzeichnet, dass** das mindestens eine Bewegungsbegrenzungsglied (92) eine in Umfangsrichtung wirkende Anschlageinrichtung (96) umfasst.

15. Wirbelsäulenstabilisierungssystem nach Anspruch 14, **gekennzeichnet durch** ein Verbindungselement (18) nach einem der Ansprüche 2 bis 13.

## Claims

1. Connecting element (18) for a stabilization system (10) for the vertebral column, comprising a first attachment section (20) for fixing to a first bone fixation device (12), a second attachment section (22) for fixing to a second bone fixation device (14), and an at least partially flexible intermediate section (24) arranged or formed between the first and second attachment sections (20, 22), said intermediate section (24) having at least one recess (66, 68) which is open at the sides in a direction transverse to a longitudinal axis (40) defined by the intermediate section (24), wherein at least one movement limiting member (92) is provided, which comprises a filling body (94) and at least one stop device (96) acting in a direction parallel or substantially parallel to the longitudinal axis (40), and wherein the filling body (94), in a normal position in which no external forces act on the intermediate section (24), engages at least partially in the at least one recess (66, 68), **characterized in that** the at least one movement limiting member (92) comprises a stop device (96) acting in a circumferential direction.

2. Connecting element in accordance with claim 1, **characterized in that** the stop device (96) comprises at least one stop (132, 134, 136, 138) which is arranged on, formed on and/or protruding from the filling body (94), which acts in a direction parallel or substantially parallel to the longitudinal axis (40) and/or in the circumferential direction.

3. Connecting element in accordance with any one of the preceding claims, **characterized in that** a plurality of movement limiting members (92) are provided and **in that** stop devices (96) of neighboring movement limiting members (92), in the normal position, lie at least partially in a noncontacting manner opposite each other and, in a position deflected from the normal position, lie against each other.

4. Connecting element in accordance with any one of the preceding claims, **characterized in that** each movement limiting member (92) comprises a stop device (96) with two stops (136, 138) facing in opposite directions.

5. Connecting element in accordance with any one of the preceding claims, **characterized in that** the at least one movement limiting member (92) and/or the intermediate section (24) is formed mirror-symmetrically in relation to a mirror plane (64) containing the longitudinal axis (40).

6. Connecting element in accordance with any one of the preceding claims, **characterized by** an anti-twisting device (150) for preventing twisting of the intermediate section (24) about the longitudinal axis (40).

7. Connecting element in accordance with any one of the preceding claims, **characterized in that** the at least one recess (66, 68), in the normal position, comprises an insertion opening (70, 72) facing transversely to and away from the longitudinal axis (40) for inserting the filling body (94) in a direction transverse to and towards the longitudinal axis (40).

8. Connecting element in accordance with claim 7, **characterized in that** the movement limiting member (92) comprises a closing element (102) for closing the insertion opening (70, 72).

9. Connecting element in accordance with any one of the preceding claims, **characterized in that** the at least one movement limiting member (92) comprises at least one side closure element (108) for at least partially closing the at least one recess (66, 68) at the sides.

10. Connecting element in accordance with claim 9, **characterized in that** the at least one side closure element (108) is formed so as to protrude from the side of the filling body (94).

11. Connecting element in accordance with claims 9 and 10, **characterized in that** the stop device (96) comprises at least one stop (132, 134, 136, 138) which is arranged on, formed on and/or protruding from the filling body (94), which acts in a direction parallel or substantially parallel to the longitudinal axis (40) and in a circumferential direction, and **in that** the at least one side closure element (108) comprises or carries at least one of the stops (136, 138) acting in a direction parallel or substantially parallel to the longitudinal axis (40) or in the circumferential direction.

12. Connecting element in accordance with any one of the preceding claims, **characterized in that** a stiffness of the intermediate section (24) has a value ranging from about 30 N/mm to about 150 N/mm.

13. Connecting element in accordance with any one of the preceding claims, **characterized by** an outside surface (80) which is machined at least partially by a blasting process.

14. Stabilization system (10) for the vertebral column, comprising at least one first bone fixation device (12), at least one second bone fixation device (14), and a connecting element (18), said connecting element (18) comprising a first attachment section (20) for fixing to the at least one first bone fixation device (12), a second attachment section (22) for fixing to the at least one second bone fixation device (14), and an at least partially flexible intermediate section (24) arranged or formed between the first and second attachment sections (20, 22), said intermediate section (24) having at least one recess (66, 68) which is open at the sides transversely to a longitudinal axis (40) defined by the intermediate section (24), wherein at least one movement limiting member (92) is provided, which comprises a filling body (94) and at least one stop device (96) acting in a direction parallel or substantially parallel to the longitudinal axis (40), and wherein the filling body (94), in a normal position in which no external forces act on the intermediate section (24), engages at least partially in the at least one recess (66, 68), **characterized in that** the at least one movement limiting member (92) comprises a stop device (96) acting in a circumferential direction.

15. Stabilization system for the vertebral column in accordance with claim 14, **characterized by** a connecting element in accordance with any one of claims 2 to 13.

## Revendications

1. Elément de liaison (18) pour un système de stabilisation de la colonne vertébrale (10) comprenant une première partie de fixation (20) à fixer à un premier dispositif de fixation osseuse (12), une deuxième partie de fixation (22) à fixer à un deuxième dispositif de fixation osseuse (14) et une partie intermédiaire (24), au moins en partie flexible, agencée ou formée entre la première et la deuxième partie de fixation (20, 22), laquelle partie intermédiaire (24) présente au moins un évidement (66, 68) ouvert latéralement dans une direction transversale à un axe longitudinal (40) défini par la partie intermédiaire (24), au moins un organe de limitation de mouvement (92) étant prévu, lequel comporte un corps de remplissage (94) et au moins un dispositif de butée (96) agissant parallèlement ou sensiblement parallèlement à l'axe longitudinal (40), et le corps de remplissage (94), dans une position de base dans laquelle aucune force extérieure n'agit sur la partie intermédiaire (24), s'insérant au moins en partie dans l'évidement ou les évidements (66, 68), **caractérisé en ce que** l'organe ou les organes de limitation de mouvement (92) comportent un dispositif de butée (96) agissant dans la direction périphérique.

2. Elément de liaison selon la revendication 1, **caractérisé en ce que** le dispositif de butée (96) comporte au moins une butée (132, 134, 136, 138) agencée, formée sur le corps de remplissage (94) et/ou faisant saillie de celui-ci, laquelle agit parallèlement ou sensiblement parallèlement à l'axe longitudinal (40) et dans la direction périphérique.

3. Elément de liaison selon l'une quelconque des revendications précédentes, **caractérisé en ce que** plusieurs organes de limitation de mouvement (92) sont prévus et **en ce que** les dispositifs de butée (96) d'organes de limitation de mouvement (92) voisins, dans la position de base, se font face au moins en partie sans contact et s'appliquent les uns contre les autres dans une position déviée de la position de base.

4. Elément de liaison selon l'une quelconque des revendications précédentes, **caractérisé en ce que** chaque organe de limitation de mouvement (92) comporte un dispositif de butée (96) pourvu de deux butées (136, 138) orientées dans des directions opposées.

5. Elément de liaison selon l'une quelconque des revendications précédentes, **caractérisé en ce que** l'organe ou les organes de limitation de mouvement (92) et/ou la partie intermédiaire (24) sont en symétrie spéculaire par rapport à un plan spéculaire (64) contenant l'axe longitudinal (40).

6. Elément de liaison selon l'une quelconque des revendications précédentes, **caractérisé par** un dispositif anti-torsion (150) destiné à empêcher une torsion de la partie intermédiaire (24) autour de l'axe longitudinal (40).

7. Elément de liaison selon l'une quelconque des revendications précédentes, **caractérisé en ce que** l'évidement ou les évidements (66, 68) présentent, dans la position de base, une ouverture d'introduction (70, 72) orientée transversalement à l'axe longitudinal (40) et à distance de celui-ci, pour l'introduction du corps de remplissage (94) dans une direction transversale à l'axe longitudinal (40) et vers celui-ci.

8. Elément de liaison selon la revendication 7, **caractérisé en ce que** l'organe de limitation de mouvement (92) comporte un élément de fermeture (102) pour l'obturation de l'ouverture d'introduction (70, 72).

9. Elément de liaison selon l'une quelconque des revendications précédentes, **caractérisé en ce que** l'organe ou les organes de limitation de mouvement (92) comportent au moins un élément d'obturation latérale (108) pour l'obturation au moins en partie latérale de l'évidement ou des évidements (66, 68).

10. Elément de liaison selon la revendication 9, **caractérisé en ce que** l'élément ou les éléments d'obturation latérale (108) font saillie latéralement du corps de remplissage (94).

11. Elément de liaison selon l'une quelconque des revendications 9 et 10, **caractérisé en ce que** le dispositif de butée (96) comporte au moins une butée (132, 134, 136, 138) agencée, formée sur le corps de remplissage (94) et/ou faisant saillie de celui-ci, laquelle agit parallèlement ou sensiblement parallèlement à l'axe longitudinal (40) et dans la direction périphérique et **en ce que** l'élément ou les éléments d'obturation latérale (108) comportent ou portent au moins une des butées (136, 138) agissant parallèlement ou sensiblement parallèlement à l'axe longitudinal (40) ou dans la direction périphérique.

12. Elément de liaison selon l'une quelconque des revendications précédentes, **caractérisé en ce qu'**une rigidité de la partie intermédiaire (24) présente une valeur dans une plage allant de sensiblement 30 N/mm à sensiblement 150 N/mm.

13. Elément de liaison selon l'une quelconque des revendications précédentes, **caractérisé par** une surface extérieure (80) usinée au moins en partie par un procédé de sablage.

14. Système de stabilisation de la colonne vertébrale (10) comprenant au moins un premier dispositif de fixation osseuse (12), au moins un deuxième dispositif de fixation osseuse (14) et un élément de liaison (18), lequel élément de liaison (18) comporte une première partie de fixation (20) à fixer au ou aux premiers dispositifs de fixation osseuse (12), une deuxième partie de fixation (22) à fixer au ou aux deuxièmes dispositifs de fixation osseuse (14) et une partie intermédiaire (24) au moins en partie flexible, agencée ou formée entre la première et la deuxième partie de fixation (20, 22), laquelle partie intermédiaire (24) présente au moins un évidement (66, 68) ouvert latéralement transversalement à un axe longitudinal (40) défini par la partie intermédiaire (24), au moins un organe de limitation de mouvement (92) étant prévu, lequel comporte un corps de remplissage (94) et au moins un dispositif de butée (96) agissant parallèlement ou sensiblement parallèlement à l'axe longitudinal (40), et le corps de remplissage (94), dans une position de base dans laquelle aucune force extérieure n'agit sur la partie intermédiaire (24), s'insérant au moins en partie dans l'évidement ou les évidements (66, 68), **caractérisé en ce que** l'organe ou les organes de limitation de mouvement (92) comportent un dispositif de butée (96) agissant dans la direction périphérique.

15. Système de stabilisation de la colonne vertébrale selon la revendication 14, **caractérisé par** un élément de liaison (18) selon l'une quelconque des revendications 2 à 13.
